# EUROPÄISCHE PATENTSCHRIFT

(11) **EP 0 760 743 B1**
(45) Veröffentlichungstag und Bekanntmachung des Hinweises auf die Patenterteilung: **18.04.2001**
(21) Anmeldenummer: 95920847.1
(22) Anmeldetag: 20.05.1995
(51) Int. Cl.: B32B 5/18, A61F 13/00, A61L 15/00, A01G 31/00, H01B 7/28

(54) **SCHICHTFÖRMIG AUFGEBAUTER KÖRPER ZUR ABSORPTION VON FLÜSSIGKEITEN**
LAYERED ELEMENT FOR THE ABSORPTION OF LIQUIDS
CORPS CONSTITUES DE COUCHES DESTINES A L'ABSORPTION DE LIQUIDES

(30) Priorität: 26.05.1994 DE 4418319
(43) Veröffentlichungstag der Anmeldung: 12.03.1997
(73) Patentinhaber: STOCKHAUSEN GmbH & Co. KG, 47805 Krefeld (DE)
(72) Erfinder: BRÜGGEMANN, Helmut, D-47829 Krefeld (DE); DAHMEN, Kurt, D-41239 Mönchengladbach (DE)
(74) Vertreter: Wolff, Felix, Dr.
(86) Internationale Anmeldenummer: EP9501926
(87) Internationale Veröffentlichungsnummer: WO9532860

(56) Entgegenhaltungen:
- EP-A- 0 427 219
- EP-A- 0 577 233
- WO-A-87/03168
- DE-A- 4 233 289
- US-A- 4 715 918
- US-A- 5 175 046

## Beschreibung

Die Erfindung betrifft Wasser und wäßrige Flüssigkeiten absorbierende Körper, die aus geschäumten Kunststoff- und Latexschichten und superabsorbierenden Polymeren bestehen, sowie ein Herstellungsverfahren für diese Körper und deren Verwendung als Absorptionsmittel, insbesondere im Hygienebereich zur Aufnahme von Körperflüssigkeiten, wie Blut, Schweiß, Urin und anderen flüssigen Ausscheidungen. Weiterhin betrifft die Erfindung die Verwendung der Körper als Komponenten in Wundabdeckungen, in Verpackungs- und Isolationsmitteln, in Textilien für Bekleidungs- und Reinigungszwecke, sowie die Verwendung im Bereich der Pflanzenzucht.

Schichtförmig aufgebaute Körper mit der Fähigkeit wäßrige Flüssigkeiten aufzunehmen sind bekannt. Die US 4 000 028 beschreibt Körper aus Latexschaum und Cellulose-Fluff, die jedoch keine superabsorbierenden Polymerisate enthalten, so daß sie eine sehr begrenzte Aufnahmekapazität für Flüssigkeiten besitzen.

In US 5 128 082 werden absorbierende Körper beschrieben, die aus Mischungen von Fluff-Materialien und superabsorbierenden Polymerisaten sowie einem umgebenden, jeweils die äußere Schicht bildenden Latex hergestellt werden. Der Kontakt zwischen Polymerisat und Latex wird weitgehend durch den Fluff-Anteil verhindert. Der Polymerisatanteil in diesen Körpern ist ungleichmäßig verteilt, was zu den bekannten Schwierigkeiten bei der Aufnahme von Flüssigkeiten und den damit verbunden Nachteilen in Bezug auf den Tragekomfort dieser Hygieneartikel führt.

In EP 0 212 618 B1 werden Windelkonstruktionen beschrieben bei denen zur Vermeidung solcher Nachteile die Polymerisate mit bestimmter Korngrößenverteilung unter Anwendung eines Gradienten in einer Cellulosefaserschicht verteilt werden. Solche Konstruktionen sind jedoch nicht ausreichend stabil, insbesondere ändert sich die Verteilung der Materialien beim Transport.

Das Mischen superabsorbierender Polymerisate mit wasserhaltigen Latexschäumen führt normalerweise dazu, daß die Schäume unter Wasserentzug zusammenbrechen, wobei die offenzellige Struktur zerstört wird, so daß nachfolgend nur die an der Oberfläche befindlichen Polymerisatpartikel in der Lage sind, Flüssigkeiten aufzunehmen.

Nach EP 0 427219 A2 sind deshalb Mischungen aus superabsorbierenden Polymeren und Latexschäumen bekannt, die dadurch erhalten werden, daß die Polymerisate als Pulverspray in den aufgeschäumten Latex eingebracht werden. Die Verfahrensweise gestattet keinen definierten Aufbau solcher Körper, insbesondere ist eine genaue Verteilung der Polymerisate nicht möglich.

Aus EP 0 577 233 A1 ist die Verwendung eines Bandes als Bestandteil der Isolation von Stromkabeln bekannt, das aus einer Vliesstoffschicht und einer Schaumstoffschicht besteht und das Teilchen eines Quellpulvers, die im Bereich der Vliesstoffschicht verankert sind, enthält.

Das Patent US 4 649 164 beschreibt geschäumte wasserabsorbierende Materialien, die aus CO₂ - freisetzenden Blähmitteln und Acrylat-(Meth)acrylsäure-Latices gebildet werden, wobei der geschäumte Latex selbst das Absorbermaterial darstellt. Aufgrund des hydrophoben Charakters der Acrylatkomponente ist die Aufnahmefähigkeit dieser Schäume gegenüber den bekannten Superabsorbern begrenzt.

Ebenso sind aus DE 42 42 015 A1 bioverträgliche, als Wundverband verwendbare, offenporige Polyurethanschäume mit Guar gum als eingelagertes Hydrogel bekannt, wobei die Gelkomponente bei der Herstellung in situ eingeschäumt wird. Die Wasseraufnahmekapazität dieser Produkte soll auf einen Wert unterhalb des dreifachen des Ausgangsgewichts begrenzt bleiben.

In EP 0 340 945 A1 werden Mischungen von Elastomeren und wasserquellbaren Hydrocolloiden, die kationisch und vorzugsweise Chitosansalze sind zur Verwendung als Wundabdeckung mit Absorptionswerten von wenigstens 180 Gew.% beschrieben, wobei die Colloidpartikel regellos im Elastomeren eingebunden sind und die Aufnahmefähigkeit für wäßrige Flüssigkeiten ebenfalls begrenzt ist.

In ähnlicher Weise sind aus DE 42 33 289 A1 hydrophile Polyurethanschaumgele bekannt, die aus Mischungen von Polyolen, Diisocyanaten und superabsorbierenden Polymerisaten hergestellt werden, wobei das superabsorbierende Polymerisat durch das herstellungsbedingte Mischen der Komponenten gleichmäßig im Schaum eingebunden ist. Die Produkte werden als Wundauflagen mit definiertem Haftverhalten eingesetzt.

Weiterhin sind nach US 5,149,335 Absorberkonstruktionen bekannt, die superabsorbierende, pulverförmige Polymerisate in faserhaltigen aneinanderliegenden zellenförmigen Strukturen dieser Konstruktionen enthalten, in denen aber das Polymerisat in dichter und unfixierter Packungsanordnung vorliegt.

In der US-PS 5,175,046 wird ein Schichtstoff beschrieben, der auf einer porösen Trägerschicht, die ein Schaumstoff sein kann, diskrete superabsorbierende Elemente aufweist, die in einem diskontinuierlichen Muster reibschlüssig an der Trägerschicht befestigt sind. Das einzelne superabsorbierende Element besteht aus einem superabsorbierenden Polymer und einem Substrat, das ebenfalls ein Schaumstoff sein kann. Das superabsorbierende Element wird hergestellt entweder durch Beschichtung des Substrats mit einem hydrophilen Monomeren, das danach polymerisiert und vernetzt wird oder durch Aufbringen eines handelsüblichen superabsorbierenden Polymerpulvers auf das vorbefeuchtete Substrat mit nachfolgender Trocknung. Die Verankerung der superabsorbierenden Elemente auf der Trägerschicht erfolgt durch Reibschluß und Verwirren der einzelnen Fasern des bevorzugt eingesetzten Faservlieses.

Die WO 87/03168 beschreibt einen Träger für die Kultivierung von Pflanzen außerhalb der Erde, der aus einer wasserdichten Umhüllung besteht, in der eine Schicht von Superabsorberteilchen gleichförmig auf einer Trägerschicht, die die Diffusion von wäßrigen Flüssigkeiten ermöglicht und aus natürlichen oder synthetischen Faserstrukturen oder auch aus Schaumstoff bestehen kann, bevorzugt jedoch Cellulosewatte ist, fixiert ist. Die Fixierung des Superabsorbers erfolgt im Fall von Cellulosewatte als Trägerschicht durch vorherige Anfeuchtung der Cellulosewatte, im Fall von Polyurethanschaum als Trägerschicht wird die Fixierung der Superabsorberteilchen bevorzugt mittels eines Klebstoffs erzielt, der in Pulverform vor der Ablage der Superabsorberteilchen auf die Trägerschicht aufgebracht wird.

In der US-PS 4,715,918 wird ein Schichtstoff beschrieben, der auf einer undurchlässigen Rückenschicht in voneinander getrennten Taschen einen Superabsorber enthält. Die Taschen, in denen der Superabsorber in dichter und unfixierter Packung gelagert ist, werden durch eine permeable Schicht abgedeckt, oberhalb der eine rasch Flüssigkeit absorbierende Schicht z. B. aus Cellulosefluff angeordnet ist, oberhalb der wiederum eine durchlässige Schicht angeordnet ist. Die Taschen, in denen der Superabsorber gelagert ist, werden durch Heißversiegeln der Trägerfolie (Rückenschicht) mit der Abdeckfolie (durchlässige Schicht) gebildet, wobei die undurchlässige Rückenschicht in der Regel ein Film aus einem heiß siegelbaren Polymeren, wie Polypropylen oder Polyethylen ist. Zwar kann die undurchlässige Rückenschicht auch ein geschäumtes Vlies sein, was jedoch wegen der geförderten Undurchlässigkeit entweder geschlossenporig sein muß oder mit einer weiteren undurchlässigen Schicht kaschiert sein muß.

Es bestand daher die Aufgabe, einen Körper auf der Basis von schichtförmig konstruierten Absorptionsmaterialien für Wasser und wäßrige Flüssigkeiten bereitzustellen, der die geschilderten Nachteile vermeidet.

Die Aufgabe konnte durch einen schichtförmigen Körper aus mindestens einer offenporigen Kunststoff- und/oder Latexschaumschicht und mindestens einer aus partikelförmigem, superabsorbierenden Polymerisat gebildeten Schicht gelöst werden, wobei der schichtförmige Körper die Menge des superabsorbierenden Polymerisats in bestimmter Verteilung und fixiert an der Grenzfläche der Schaumschicht enthält.

Gegenstand der Erfindung ist demnach ein schichtförmiger aus einer oder mehreren Kunststoffschaumschichten und/oder Latexschaumschichten und partikelförmigen superabsorbierenden Polymerisaten bestehender Körper zur Absorption von Wasser und wäßrigen Flüssigkeiten, der dadurch gekennzeichnet ist, daß direkt auf, zwischen oder unter den geschäumten Kunststoff- und/oder geschäumten Latexschichten das superabsorbierende Polymerisat in mengenmäßig und/oder örtlich vorgegebener und fixierter flächenmäßiger Anordnung enthalten ist und das Mengenverhältnis von geschäumtem Kunststoff- und/oder geschäumter Latexschicht zum superabsorbierenden Polymerisat 1 : 500 bis 50 : 1, vorzugsweise 1 : 50 bis 25 : 1 und besonders bevorzugt 1 : 5 bis 10 : 1 beträgt. Der schichtförmige Absorberkörper kann starr oder flexibel sein.

Überraschenderweise bleibt beim Absorptionsvorgang unter Verwendung des erfindungsgemäßen schichtförmigen Körpers trotz des direkten Kontakts zwischen Kunststoff bzw. Latex und Polymerisat das Quellvermögen des Polymerisats unbeeinträchtigt, sodaß die Aufnahmekapazität des Polymerisats für wäßrige Flüssigkeiten auch in der gewählten Anordnung erhalten bleibt.

Der erfindungsgemäße schichtförmige Absorberkörper weist bevorzugt bei Verwendung einer 0,9%igen NaCl-Lösung eine Retention von mindestens 0,1 Liter/m² Oberfläche, eine maximale Aufnahme von mindestens 0,1 Liter/m² Oberfläche und eine Absorption unter Belastung (AUL) von mindestens 2 g/g bei 0,021 Pa auf. Gegenstand der Erfindung ist weiter ein Herstellungsverfahren sowie die Verwendung der erfindungsgemäßen schichtförmigen Absorberkörper. Das Herstellungsverfahren ist
dadurch gekennzeichnet, daß mindestens
a) ein Kunststoffschaum und/oder Latexschaum mit einem Litergewicht von 50 bis 1000 g/l erzeugt und
   der vorzugsweise offenporige Schaum flächenförmig in einer Schichtdicke von 1 µm bis 100.000 µm, vorzugsweise von 10 µm bis 10.000 µm und besonders bevorzugt von 200 µm bis 5.000 µm verteilt wird,
b) das superabsorbierende, partikelförmige Polymerisat in einem Mengenverhältnis von geschäumtem Kunststoff- und/oder geschäumter Latexschicht zum superabsorbierenden Polymerisat von 1 : 500 bis 50:1, vorzugsweise 1 : 50 bis 25 :1 und besonders bevorzugt 1: 5 bis 10:1 gegebenenfalls unter Verwendung mindestens einer Schablone, einer Lochscheibe und/oder eines Siebes in bestimmter mengen- und flächenmaßiger Verteilung auf den flächenförmig verteilten Schaum aufgebracht, durch Wärmebehandlung fixiert wird, wobei gegebenenfalls der Verfahrensschritt a) und/oder b) in beliebiger Reihenfolge wiederholt wird und abschließend eine Wärmebehandlung unter Vernetzung der geschäumten Schichten erfolgt.

Die Herstellung des erfindungsgemaßen absorbierenden Körpers geschieht unter Verwendung bekannter Rohstoffe.
Als Grundlage für die Kunststoff- oder Latexschaumschicht können handelsübliche Kunststoffe und/oder Latexdispersionen für Hart-, halbharte, elastische und weichelastische Schaumkunststoffe aus Polystyrol, Styrolcopolymeren, Hart- und Weich-PVC, Polycarbonaten, Polycarbonimiden, Polymethacrylimiden, Polyamiden, Polystyrol-Butadien Polymerisaten, sowie Phenol- und Harnstoffharzen, vorzugsweise aus wenigstens zwei Monomeren gebildete Copolymerisate aus (Meth)acrylaten, Styrol, Butadien, Vinylacetat und den zu polymerisierten Vinylalkoholeinheiten ganz oder teilweise verseiften Vinylacetaten eingesetzt werden.
Der Kunststoffschaum und/oder Latexschaum wird mit bekannten Mitteln, beispielsweise durch starkes Rühren oder Mixen unter Vermischung mit Luft in der Regel unter Zusatz von Schaumhilfsmitteln erzeugt.

Die Art und Menge des eingesetzten Kunststoff- oder Latexmaterials bestimmt in bekannter Weise die mechanischen Eigenschaften der erfindungsgemäßen Körper, wie z.B. den Grad der Flexibilität sowie das Oberflächenverhalten der Konstruktionen.

Es wurde festgestellt, daß sich durch Mischen der Kunststoff- und Latexmaterialien die Eigenschaften beeinflussen lassen. Die Aufnahmegeschwindigkeit von Wasser oder wäßrige Flüssigkeiten wird wesentlich von der Art des verwendeten Kunststoff-und/oder Latexmaterials bestimmt. Insbesondere wurde überraschenderweise festgestellt, daß durch Mischen dieser Komponenten in bestimmten Fällen die Aufnahmegeschwindigkeit so verbessert werden kann, daß sie über dem Wert der absorbierenden Körper liegt, die nur aus einer Kunststoff- oder Latexkomponente gebildet werden.

Ebenso findet überraschenderweise eine starke Beeinflussung der Aufnahmegeschwindigkeit durch die Art und Menge sowie die Verteilung der zusätzlich eingesetzten Füllstoffmaterialien statt.
Geeignete Füllmaterialien sind Kreiden, Bentonite, Kieselgele und Kieselsäure, Aktivkohlen, Pigmente, wie Titandioxid und Eisenoxid, sowie natürliche und/oder synthetische Fasermaterialien, wie Viskose- und Baumwollfasern und -gewebe und/oder Polyester- und Polyamidfasern und Mischungen verschiedener Fasern oder entsprechender Gewebe. Weiterhin sind feingemahlene Kunststoffe, besonders solche von der gleichen Art wie das verwendete Kunststoff- oder Latexmaterial geeignet. Die Art, Konzentration und Verteilung des Füllmaterial kann in jeder Schaumschicht gleich oder verschieden sein. Ebenso können Mischungen verschiedener Füllstoffe verwendet werden. Die einzelne Schaumschicht kann einen Füllungsgrad von 0 bis 1000 Gew.%, bezogen auf die Menge des Kunststoffs oder Latex, bevorzugt maximal 400 Gew.% und besonders bevorzugt maximal 200 Gew.% aufweisen. Darüber hinaus können die beschriebenen Füllmaterialien auch als separate Schicht in den absorbierenden Körper eingebracht werden. Das superabsorbierende Polymerisat kann auch als Mischung mit den als Füllstoff genannten Materialien aufgebracht werden.
Die Verwendung der Kunststoff- oder Latexdispersion kann darüber hinaus unter Verwendung weiterer Zusätze, wie Bläh- und Schäumungsmitteln, Schaumstabilisatoren, Vernetzungs- und Vulkanisationsmitteln erfolgen; die Verarbeitungsbedingungen zur Herstellung und Stabilisierung solcher Schäume sind bekannt.

Der Latexschaum kann in geometrisch verschiedenen Formen gebildet werden, wobei die Erzeugung einer offenporigen Schaumschicht mit beliebiger Dicke bevorzugt ist. Hierbei können, wie in US 4 000 028 angegeben, zur Herstellung abtrennbare Flächen von Hilfsträgern, wie Metallbänder und Folien, Siliconpapier, Glasfasern, Glasflächen oder textile Gewebe, hilfsweise verwendet werden oder es können erfindungsgemäß vorzugsweise Flächen von Materialien, wie flüssigkeitsdurchlässige und -undurchlässige Kunststoffolien und Vliese, Zellstoff- oder Papierschichten oder textile Gewebe als Basis eingesetzt werden, die Bestandteil des absorbierenden Körpers werden.

Die Oberfläche des erfindungsgemäßen, schichtförmig aufgebauten Körpers kann teilweise, vorzugsweise einseitig, eine für wäßrige Flüssigkeiten und Wasser undurchlässige Schicht, eine Kunststofffolie und/oder ein Gewebe, und/oder teilweise, vorzugsweise einseitig, eine Schicht aus Zellstoff, Vlies und/oder Papier und/oder ein textiles Vlies oder Gewebe enthalten.

Ebenso kann die Basisschicht aus dem nicht aufgeschäumten Kunststoff- oder Latexmaterial bestehen, die als verdickte Dispersion ausgestrichen und zu einer homogenen, flüssigkeitsundurchlässigen Schicht vulkanisiert wird. Sofern nicht andere Materialien, wie Füllstoffe oder das partikelfömige Polymerisat selbst hierzu verwendet werden, können die als Basis genannten Materialien oder eine Schaumschicht zur Abdeckung der zuletzt aufgebrachten Schicht dienen.

Die partikelförmigen, superabsorbierenden Polymerisate können erfindungsgemäß aus wasserunlöslichen, wasserquellbaren Polymerisaten oder Copolymerisaten aus Mono mereneinheiten von (Meth)acrylsäure, Maleinsäure, Itaconsäure sowie deren Anhydriden und Salzen, Fumarsäure und deren Salzen, insbesondere deren Alkali-, Erdalkali und Amoniumsalzen, (Meth)acrylamid, (Meth)acrylnitril und Vinylacetat und dessen Hydrolyseprodukten, Vinylpyrrolidon, Vinylpyrridin, Vinylsulfonsäure und deren Estern und Amiden sowie von N-Alkyl- und NN-Dialkyl- substituierten Estern und/oder Amiden der (Meth)acrylsäure und deren Salzen und/oder deren quartären Amoniumverbindungen bestehen. Ebenso sind natürliche wasserquellbare Polymerisate, wie Carboxymethylcellulose, Hydroxyethylcellulose, Guarkernmehl, Xanthane, Alginate, Stärke und deren Derivate sowie Pfropf-Polymerisate aus diesen Stoffen und den genannten Monomeren oder Mischungen der zuvor genannten Polymerisate mit diesen Stoffen verwendbar.

Das partikelförmige, superabsorbierende Polymerisat wird auf die zuvor hergestellte Oberfläche der Kunststoff- oder Latexschaumschicht in verteilter Form als Pulver mit einer Kornverteilung von 1 µm bis 20.000 µm aufgebracht. Dies kann beispielsweise durch Aufstreuen des Pulvers aus geeigneten Behältern oder mittels geeigneter Vorrichtungen erfolgen.

Die Korngröße der Pulver ist von der Verwendung der absorbierenden Körper abhängig. Im Hygienebereich werden Pulver mit Korngrößen zwischen 50 µm und 1.000 µm bevorzugt, während bei der Verwendung zur Kabelisolierung ein Bereich unter 400 µm gewählt wird.
Die erfindungsgemäßen Körper enthalten Feinstkornanteile der superabsorbierenden Polymerisate nur in sehr geringer Menge, wobei gerade diese Partikel im Oberflächenbereich der Schaumschicht fixiert sind. Insbesondere wird eine weitere Bildung feinster Polymerisatpartikel bei der Handhabung der erfindungsgemäßen Körper aufgrund mechanischer Vorgänge vermieden.

In einer speziellen Ausführungsform kann die Menge und die Verteilung des Pulvers bezogen auf die Flächeneinheit so erfolgen, daß nur bestimmte Oberflächenbereiche der Schaumschicht belegt und/oder die Flächen mit unterschiedlichen Mengen belegt werden. Hierbei kann die Auftragung unter Verwendung von Schablonen, Lochplatten, Sieben oder geeigneten Kombinationen daraus, gegebenenfalls unter Klassierung der Partikelgröße, der Polymerisate erfolgen. Beispielsweise kann durch die Auftragung von Pulvern in feinkörniger Form eine flüssigkeitsblockierende Schicht oder durch Auftragen grobkörniger Polymerisatanteile entgegengesetzt eine verbesserte Verteilung der Flüssigkeit erreicht werden.

Die Menge, Korngröße und Verteilung des partikelförmigen, superabsorbierenden Polymerisats auf den einzelnen geschäumten Kunststoffschichten und/oder geschäumten Latexschichten kann verschieden sein.

Die Flächenbelegung der Kunststoff- und/oder Latexschaumfläche liegt im Bereich von 0,1 g bis 500 g des partikelförmigen, superabsorbierden Polymerisats, bezogen auf einen m² der geschäumten Oberfläche des Körpers, vorzugsweise von 10 bis 300 g/m² und besonders bevorzugt von 50 bis 200 g/m².

Der Anteil des superabsorbierenden Polymerisats an der Gesamtkonstruktion des absorbierenden Körpers beträgt 15 - 99 Gew.%, vorzugsweise 40 - 90 Gew.% und besonders bevorzugt 50 - 80 Gew.%.

Die Herstellung des absorbierenden Körpers erfolgt durch Auftragen einer oder mehrerer Kunststoff- und/oder Latexschaumschichten im Wechsel mit dem Auftrag des partikelförmigen Superabsorbers auf die bereits erzeugte Schicht. Hierbei kann jeweils nach dem Aufbringen des Polymerisats die Kunststoff- bzw. Latexschaumschicht zur Vereinfachung bei der weiteren Verarbeitung unter geeigneten, bekannten Bedingungen, wie durch einfaches oder mehrfaches Erhitzen, beispielsweise im IR-Feld, durch Behandlung im UV-Feld der Schaum ganz oder teilweise vulkanisiert werden. Bei unterschiedlichem Aufbringen der Polymerisatpartikel auf bzw. in einzelnen Schichten werden insgesamt Körper hergestellt, in denen das absorbierende Polymerisat mit einem bestimmten Gradienten verteilt ist. Abschließend erfolgt eine Vulkanisation zur vollständigen Vernetzung der Kunststoff- oder Latexschichten, die mit einer zusätzlichen Trocknung des Körpers verbunden werden kann.

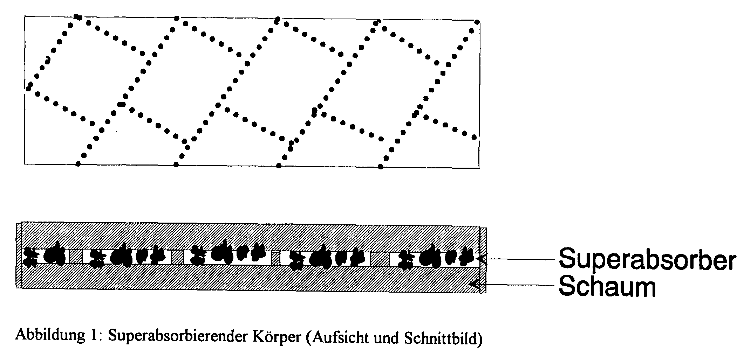

Der erfindungsgemäße Körper kann gegebenenfalls abschließend mit einem Kalander und/oder mit einer Prägewalze bearbeitet werden.
Ein bevorzugtes Beispiel des erfindungsgemäßen absorbierenden Körpers zeigt die Abbildung 1.

Die erfindungsgemäßen Körper sind zur Absorption von Wasser und wäßrigen Flüssigkeiten verschiedenster Art verwendbar. Sie finden insbesondere direkt oder als Komponente oder als Zusatz von Artikeln für den Hygiene- und Pflegebereich in Windeln, Tampons und in Inkontinenzartikeln sowie in Sanitärartikeln zur Wundabdeckung Verwendung. Weiterhin sind die absorbierenden Körper als Wasser und wäßrige Lösungen speicherndes Pflanzenwuchsmedium, zur Lagerung und zum Transport von Pflanzen und Pflanzenteilen, zur Isolation von Rohren und Leitungen, insbesondere für elektrische und lichtleitende Kabel und als Bestandteil von Bauteilen, beispielsweise zur Isolation von Außenmauern und als Verpackungsmittel oder -komponente für Handelswaren, insbesondere für Lebensmittel und Getränke geeignet. Weiterhin können sie zur Verbesserung des Tragekomforts in Bekleidungsstücke eingearbeitet werden.

Die Eigenschaften der erfindungsgemäßen Wasser und wäßrige Flüssigkeiten absorbierenden Körper können durch die im folgenden dargestellten Testmethoden erfaßt werden.

### Testmethoden:

A. Teebeuteltest (TBT)
   Zur Bestimmung des Absorptionsvermögens wurde der TBT durchgeführt. Als Prüflösung wurde (soweit nicht anders erwähnt) eine 0,9 %-ige NaCl - Lösung verwendet.
   Aus dem absorbierenden Körper wird entsprechend der durch die Herstellung vorgegebenen Schicht des superabsorbierenden Polymeren ein Stück Material ausgestanzt, das 0,2 g des superabsorbierenden Polymeren (SAP) enthält. Dieses Stück wird in einem Teebeutel eingewogen. Anschließend wird der Teebeutel für 10 Minuten in die Testlösung gelegt. Nach 5-minütiger Abtropfzeit wurde der Teebeutel ausgewogen, anschließend wurde der Teebeutel in einer Zentrifuge (handelsübliche Wäscheschleuder, 1400 Upm) abgeschleudert. Danach wurde wiederum ausgewogen. Die Flüssigkeitsaufnahme wird entweder auf 1 g des Körpers, auf 1 g des eingesetzten SAP oder auf 1 m² des Körpers berechnet.
B. Absorption under Load (AUL)
   Um das Flüssigkeitsaufnahmevermögen unter Druck zu bestimmen, wurde die "Absorption under Load" (AUL), wie in der EP-A-0 339 461 beschrieben, bestimmt, wobei abweichend von dieser Vorschrift ein kreisrundes Stück des superabsorbierenden Körpers von der Größe des Innendurchmessers des AUL - Tiegels als Prüfsubstanz eingesetzt wurde. Die Flüssigkeitsaufnahme wurde entweder auf 1 g des Körpers, auf 1 g des eingesetzten SAP oder auf 1 m² der Oberfläche des Körpers berechnet.
C. Demand-Absorbency-Test
   Zur Ermittlung einer weiteren anwendungstechnischen Eigenschaft wird die Aufnahme von Modellurin nach dem "Demand Absorbency Test" (DAT) (W.F. Schlauch, Vortrag Index 1978, Amsterdam, DE 39 17 646) durchgeführt und die Aufnahmegeschwindigkeit bestimmt. Das Meßgerät besteht aus einer Bürette, die mit der Modellurinlösung (2,0 % Harnstoff, 0,9 % NaCl, 0,1 % MgSO₄ und 0,06 % CaCl₂, aufgelöst in dest. Wasser) gefüllt ist, und einem Probetisch, der mit einer an die Meßbürette angeschlossenen Öffnung für den Modellurinaustritt vorgesehen ist. Auf den Probetisch wird ein Stück eines erfindungsgemäßen Körpers, das 1 g des SAP beinhaltet, zentrisch über der Flüssigkeitsaustritt gelegt. Anschließend wird durch leichten Druck auf den Verbindungsschlauch der Kontakt zwischen der Modellurinlösung und dem Probekörper hergestellt. Die Flüssigkeitaufnahme, abgelesen an der Bürette, wird nach jeder Minute notiert. Nachdem 50 ml der Testlösung aufgenommen wurden, wird die hierfür benötigte Zeit notiert.
   Ein zweiter Test (DATP) wird in gleicher Weise durchgeführt, jedoch mit dem Unterschied, daß das Probestück mit einem Druck ( 2,07 Pa ≙ 0,3 psi) belastet wird. Die Werte werden in gleicher Weise abgelesen und angegeben.
D. Schiefe - Ebene - Test
   Der "schiefe - Ebene - Test" zur Bestimmung der Absorptionseigenschaften der erfindungsgemäßen Körper erfolgt in Anlehnung an eine in EP 0 546 587 A1 erläuterte Testmethode.
   Ein 10 cm x 30 cm großes Stück des erfindungsgemäßen, absorbierenden Körpers wird auf einer schiefen Ebene (Neigungswinkel: 45°) in Längsrichtung befestigt. Mit einem Tropftrichter werden in der Mitte der oberen Kante des Prüfkörpers 100 ml einer 0,9 %-ige NaCl - Lösung mit einer Fließgeschwindigkeit von 4 ml/sec. aufgegeben. Die über die Oberfläche des Prüfkörpers ablaufende Flüssigkeit wird direkt in einem Auffanggefäß 1 gesammelt und gemessen. Nach 10 Min. wird die gesamte von der Oberfläche des Auffanggefäßes 1 und aus dem Prüfkörper in das Auffanggefäß 2 abgeflossene Flüssigkeitsmenge bestimmt. Der Vorgang wird solange wiederholt, bis die Aufnahmekapazität des Prüfkörpers, in der Regel nach dem 4. oder 5. Flüssigkeitsauftrag, erschöpft ist.

Als Testergebnis werden angegeben:
1) die Flüssigkeitsmenge, die über die Oberfläche abgelaufen ist, und im Auffanggefäß 1 aufgenommen wurde (OA),
2) die Zeit, bis zum Austritt der Flüssigkeit an der Unterkante des Prüfkörpers (ZA) sowie,
3) die Gesamtmenge an Flüssigkeit, die sich nach 10 Minuten in den Auffangwannen 1 und 2 gesammelt hat (AV).

Die Erfindung wird an den nachstehenden Beispielen erläutert.

Die zur Herstellung der Kunststoff- und/oder Latexschaumschichten verwendeten Produkte werden im folgenden nur mit den Handelsnamen benannt und entsprechend ihrer chemischen Zusammensetzung charakterisiert:

| | |
|---|---|
| Estekoll^{®} HL 40 | Acrylsäureester |
| Estekoll^{®} 60 | Vinylcopolymerisat |
| Estekoll^{®} SU 390 | Acrylsäureester Copolymerisat |
| Fixamin^{®} PU 603 | Polyurethan/Polyester (ohne freie Isocyanatgruppen) |
| Fixamin^{®} PU421 | Polyurethan (aliphatisch, ohne freie Isocyanatgruppen) |
| Fixamin^{®} PUK | Polyesterpolyurethan (ohne freie Isocyanatgruppen) |
| Fixamin^{®} PU 555 | Polyesterpolyurethan (ohne freie Isocyanatgruppen) |
| Sarpifan^{®} CAW | Vinylcopolymerisat (plastifiziert) |
| Sarpifan^{®} DFP | Vinylcopolymerisat (plastifiziert) |
| Sarpifan^{®} BKF | Polyvinylacetat (weichmacherfrei) |
| Sarpifan^{®} HP 79 | Vinylcopolymerisat (weichmacherfrei) |
| Sarpifan^{®} NL | Nitrillatex |
| Sarpifan^{®} U 75 | Acrylsäurecopolymerisat |
| Sarpifan^{®} VT | Acrylsäureestercopolymerisat |
| Sarpifan^{®} VB | Acrylsäureester |
| Sarpifan^{®} WRG | Acrylsäureester |
| Sarpifan^{®} VBA | Butadiencopolymerisat |
| Stokal^{®}STD | Ammoniumstearat |
| Mirox^{®}TA | Kaliumpolyacrylat |
| Lavoral^{®}LO | Fettalkoholsulfat |
| Favor^{®}SAB 922 FAF | schwach vernetztes, teilneutralisiertes Polyacrylat |
| Favor^{®}SAB 990 | schwach vernetztes, teilneutralisiertes Polyacrylat |
| Favor^{®}922 SK | schwach vernetztes, teilneutralisiertes Polyacrylat |
| Plantaren^{®}2000 CS/UP | Alkylpolyglykosid |
| Favor^{®}SXM 75 | schwach vernetztes, teilneutralisiertes Polyacrylat |
| Favor^{®}SXM 100 | schwach vernetztes, teilneutralisiertes Polyacrylat |
| Bunatex^{®}SL 3510 | Styrol/Butadien Copolymerisat |
| Bunatex^{®}SL 2810 | Styrol/Butadien Copolymerisat |
| Acronal^{®}DS 2331 X | Copolymer auf Basis Ethylacrylat |
| Kaolin^{®}W | Kreidemineral |
| Neogel^{®}V 70 ZB | Vulkanisationspaste auf Basis Schwefel |
| Neogel^{®}V 77 ZB | Vulkanisationspaste auf Basis Schwefel |
| Calcicoll^{®}W 12 | teil kristalline Kreide |
| Fixapet^{®}VNF | stickstoffhaltiger, formaldehydfreier Vernetzer |
| Vinipas^{®}LL 778/5 | Ethylen/Vinylacetat Copolymer |
| Lipolan^{®}VD 9910 | Butadien/Styrol Copolymer |
| Litex^{®}AP 4120 | Butylacrylat/Styrol Copolymer |

### Beispiele 1 - 4

80 Teile Fixamin^{®}PUK, 3 Teile Stokal^{®}STD, 5 Teile Mirox^{®}TA, 11 Teile deionisiertes Wasser und 1 Teil Lavoral^{®}LO werden gemischt und mit einem Handmixgerät auf ein Schaumlitergewicht von 250 g/l aufgeschlagen.
Der Schaum wurde auf Baumwollnessel aufgerakelt (1,5 mm Schichtdicke) und anschließend mit einem großen Überschuß von 500 g/m² des superabsorbierenden Polymerisats (SAP) bestreut. Es wurde 6 min auf 100°C im Trockenschrank erwärmt, anschließend wurden die Proben kalandert, das nicht anhaftende SAP wurde abgeschüttelt. Die Proben wurden dann abermals für 5 min auf 170°C erwärmt.

| | SAP | Teebeuteltest | |
|---|---|---|---|
| | | (max.) | (ret.) |
| | [Art] | [l/m²] | [l/m²] |
| Beispiel 1 | Favor^{®}SAB 922 FAF | 1,9 | 1,4 |
| Beispiel 2 | Favor^{®}SAB 990 | 6,8 | 4,9 |
| Beispiel 3 | Favor^{®}922 SK | 9,6 | 8,2 |
| Beispiel 4 | Carboxymethylcellulose (gemäß Bsp. 2 Nr. 50 in EP 053 8904 A2) | 3,8 | 1,5 |

### Vergleichsbeispiele 1 - 4

Es wurde entsprechend der Beispiele 1 - 4 verfahren, mit dem Unterschied, daß der Schaum mit dem SAP gemischt wurde und die so erhaltene Masse auf Baumwollnessel aufgestrichen wurde (Dicke ca. 3 mm). Anschließend wurde weiter, wie in den Beispielen 1 - 4 verfahren.
Keine der untersuchten Proben zeigte eine Aufnahme (TBT) von mehr als 0,2 l/m².

### Vergleichsbeispiele 5 und 6

20 g Caradol 48-2 (Polyol der Firma Shell), 0,2 g Tedostab (Polysiloxan der Firma Goldschmidt), 0,08 g Dibutylzinndilaurat und 0,08 g N,N-Dimethylaminoethanol werden gemischt. Zu diesem Gemisch wird Favor SXM 100 gegeben. Dann wird unter Rühren eine Mischung aus 1 g Wasser und 10 g Toluylendiisocyanant zugegeben. Die Mischung wird auf eine Polyethylenfolie gegossen. Nach 2 h ist der Polyurethanschaum ausgehärtet. Das weiße, mäßig flexible Gebilde hat eine Dicke von etwa 5 mm.

| Vergleichsbeispiel | Favor SXM 100 | TBT (ret.)* | |
|---|---|---|---|
| | | (30 Sek.) | (30 Min.) |
| | (g) | (g/g) | (g/g) |
| 5 | 15,68 | 4,5 | 12,0 |
| 6 | 7,84 | 3,1 | 9,70 |

| | | | |
|---|---|---|---|
| * Die gemessenen Werte beziehen sich auf den eingesetzten Superabsorber. | | | |

### Beispiel 5

Es wurde, wie in den Beispielen 1 - 4 beschrieben, ein Schaum bereitet. dieser Schaum wurde mit einer Schichtdicke von 1,5 mm auf ein Baumwollnesseltuch aufgetragen. Anschließend wurde ein Raster auf den Schaum gelegt. Die frei bleibenden Schaumfelder wurden mit Favor®SAB 990 bestreut (Flächenkonzentration 150 g/m² bezogen auf die Gesamtfläche). Anschließend wurde das Raster wieder abgenommen und eine Deckschicht von 1 mm des beschriebenen Latexschaums aufgebracht. Der Schaum wurde dann wie in den Beispielen 1 - 4 beschrieben behandelt.
TBT*(max.): 6,5 l/m² TBT*(ret.): 5,0 l/m²
* in diesem Falle wurde die Messung des TBT wie folgt durchgeführt: Je eine der entstandenen Taschen wurde aus dem hergestellten Körper ausgeschnitten. Diese Tasche wurde dann, ohne sie in einen Teebeutel einzuschweißen, zur Messung der Absorptionswerte verwendet.

### Beispiele 6 und 7

Die Verfahrensweise von Beispiel 5 wird wiederholt, wobei aber statt Favor SAP 990 ein vernetztes Guarkernmehl verwendet wird. Der Polymerschaum wird 30/60 min (Beispiel 6/7) bei 120°C vernetzt. Die Absorptionen wurden in Abhängigkeit von der Tauchzeit bestimmt:

| | Tauchzeit | Teebeuteltest | |
|---|---|---|---|
| | [min.] | max. | ret. |
| | | [g/g SAP] | [g/g SAP] |
| Beispiel 6 | | | |
| | 1 | 5,9 | 7,3 |
| | 10 | 15,0 | 14,9 |
| | 60 | 15,7 | 16,3 |
| | 240 | 16,7 | 17,3 |
| Beispiel 7 | 1 | 7,3 | 9,0 |
| | 10 | 13,2 | 11,1 |
| | 30 | 14,6 | 11,8 |
| | 60 | 14,6 | 13,5 |
| | 240 | 15,9 | 15,2 |

### Beispiele 8 - 10

Ein Schaum wird auf die beschriebene Weise aus 0,8 g Plantaren^{®}2000 CS/UP, 1 g Stokal^{®} SR, 0,2 g Guarkernmehl 104, 3 g Fixamin^{®}PUK, 15 g Sarpifan^{®}VBA und 10 g deionisiertes Wasser bereitet. Der Schaum wird auf ein Gesamtvolumen von 400 ml aufgeschäumt und auf eine Fläche von 0,1 m² gleichmäßig verteilt. Anschließend wird 30 g eines SAP gleichmäßig auf diese Fläche verteilt. Die so entstandene Masse wird 5 Minuten bei 160°C im Trockenschrank getrocknet. Anschließend wird die gleiche Schaumschicht auf diese Masse aufgetragen, wiederum mit derselben Menge des SAP bestreut und abermals 5 Minuten bei 160°C getrocknet. Diese Prozedur wird im Falle der Beispiele 8 und 9 noch 2 mal wiederholt. Anschließend wird eine letzte Schaumschicht aufgetragen und ohne weiteres Bestreuen der erhaltene Körper 5 Minuten bei 160°C im Trockenschrank erhitzt.

| | SAP | TBT | |
|---|---|---|---|
| | (Art) | (max.) | (ret.) |
| | | [l/m²] | [l/m²] |
| Beispiel 8 | Favor^{®}SXM 75 | 53 | 36 |
| Beispiel 9 | Favor^{®} 922 FAF | 59 | 46 |
| Beispiel 10* | Favor^{®} 922 FAF | 31 | 22 |

| | | | |
|---|---|---|---|
| * In jede Absorberschicht wurden zusätzlich 40 g/m² Polyamidfaser (NC 0261) eingearbeitet. | | | |

### Beispiel 11

0,8 g Plantaren^{®}2000 CS/UP, 0,4 g Stokal^{®} SR, 8 g deionisiertes Wasser und 13,8 g Fixamin^{®}PU 421 werden aufgeschäumt (Schaumlitergewicht ca. 300 g/1) und auf eine Metallplatte mit einer Größe von 460 cm² verteilt und anschließend für 10 Minuten auf 160°C erwärmt. Anschließend wird ein Schaum aus 0,4 g Plantaren^{®}2000 CS/UP, 0,4 g Stokal^{®}SR, 6 g deionisiertem Wasser und 4,9 g Estekoll^{®} SU 390 hergestellt (Schaumlitergewicht wie zuvor) und auf die vorhandene Schaumschicht aufgetragen. Diese Schaumschicht wird mit 13,8 g Favor^{®} SXM 75 bestreut und anschließend bei o.g. Bedingungen erhitzt. Danach wird eine weitere Schicht des zweiten Schaums aufgetragen und abermals mit dem gleichen SAP in gleicher Menge bestreut und thermisch nachbehandelt. Darauf wird wieder die gleiche Schaumschicht aufgetragen, diese jedoch nicht mit SAP bestreut. Der entstandene Körper erhält nach abermaligem Erhitzen auf 160°C (10 Minuten) eine Deckschicht aus der o.g. Rezeptur und wird erneut erhitzt. Anschließend wird der so erhaltene, weiche und flexible Körper von der Metalloberfläche abgelöst.
TBT (max./ret.): 25/16,6 [l/m²]

### Beispiele 12 - 16

Es wird aus 2 g Plantaren®2000 CS/UP, 2 g Stokal® SR, 5 g Fixamin®PUK, 26 g Sarpifan®VBA, 5 g Estekoll® 60 und 20 g deionisiertem Wasser ein Schaum mit einem Volumen von ca. 0,8 l aufgeschlagen.
Der Schaum wird in 2 Hälften geteilt. Die erste Hälfte wird auf einem Blech auf einer Fläche von 0,1 m² ausgestrichen, anschließend wird das SAP Favor® SXM 75 in definierter, in der folgenden Tabelle angegebenen Menge, gleichmäßig auf die Fläche aufgestreut. Anschließend wird diese Masse bei 160°C 5 Minuten lang getrocknet, dann mit der 2. Hälfte an aufgeschlagenem Schaum bedeckt und wiederum bei den selben Bedingungen getrocknet. Der so erhaltene Körper wird vom Blech abgelöst und auf seine Aufnahmefähigkeit hin untersucht.

| Beispiel | Menge an aufgestreutem SAP | Teebeuteltest (10') | |
|---|---|---|---|
| | | (max.) | (ret.) |
| | [g/m²] | [l/m²] | [l/m²] |
| 12 | 150 | 10,2 | 6,1 |
| 13 | 200 | 11,6 | 7,3 |
| 14 | 250 | 14,7 | 9,9 |
| 15 | 300 | 16,0 | 10,5 |
| 16 | 400 | 18,2 | 11,4 |

### Beispiel 17,18

1 g Plantaren^{®}2000 CS/UP, 1 g Stokal^{®} SR, 12 g Sarpifan^{®}VBA, 6 g Fixamin^{®}PUK und 10 g deionisiertes Wasser werden wie beschrieben aufgeschäumt und auf einem Blech auf eine Fläche von 0,1 m² ausgestichen (Fußschicht). Die Masse wurde mit einer Mischung aus 30 g Favor^{®} SAB 990 und 0,3 g Polyamidfaser bestreut und anschließend bei 150°C 5 Minuten getrocknet. Anschließend wird ein Schaum aus 0,8 g Plantaren^{®}2000 CS/PU, 0,8 g Stokal^{®} SR, 6,7 g Sarpifan^{®}VBA, 3,1 g Estekoll^{®} SU 390 und 10 g deionisiertem Wasser, aufgeschäumt auf ca. 400 ml, aufgestrichen. Diese Schicht wird ebenfalls mit den o.g. Mengen an Favor^{®} SAB 990 und Polyamidfaser bestreut und für 5 Minuten bei 150°C getrocknet. Es werden 2 weitere, gleiche Schichten aufgetragen. Danach wird auf diesem Körper eine Deckschicht entsprechend der Fußschicht aufgetragen.

In einem weiteren Versuch (Beispiel 18) wird der vorhergehende Versuch mit dem Unterschied wiederholt, daß für die Deckschicht bzw. die Fußschicht 1,5 g Fixamin^{®}PUK und 16,5 g Sarpifan^{®}VBA eingesetzt werden.

Von diesen Beispielen wurden die Absorptionen nach einer bzw. nach 10 Minuten sowie der AUL Wert bestimmt. Die Werte wurden auf die Menge an eingesetztem SAP bezogen.

| Beispiel | TBT (1 Minute) | | TBT (10 Minuten) | | AUL |
|---|---|---|---|---|---|
| | (max.) | (ret.) | (max.) | (ret.) | |
| | [g/g] | [g/g] | [g/g] | [g/g] | [g/g] |
| 17 | 20,4 | 18,9 | 41,8 | 28,0 | 24,4 |
| 18 | 19,8 | 18,2 | 44,0 | 28,8 | 25,6 |

### Vergleichsbeispiele 5,6

Die Beispiele 17 und 18 wurden ohne den Zusatz eines SAP wiederholt.
Die damit erreichten Absorptionen wurden auf 1 g des Körpers berechnet:

| Vergleichsbeispiel | TBT (10 Minuten) | | AUL |
|---|---|---|---|
| | (max.) | (ret.) | |
| | [g/g] | [g/g] | [g/g] |
| 5 | 1,2 | 0,5 | 1,3 |
| 6 | 7,3 | 0,7 | 4,0 |

### Beispiel 19-33

Zu einer Mischung von 0,8 g Plantaren^{®}2000 CS/UP, 0,8 g Stokal^{®} SR, 9,4 g Sarpifan^{®}VBA und 10 g Wasser wird eine weitere Dispersion gegeben (siehe Tabelle). Daraus wird ein Schaum hergestellt und auf 0,1 m² ausgestrichen, mit 20 g Favor^{®} SXM 75 bestreut und anschließend wird 5 Minuten bei 150°C getrocknet. Die gleiche Schaumschicht wie zuvor wird auf dem so erhaltenen Körper verstrichen, anschließend werden 10 g des genannten SAP aufgestreut und wie beschrieben getrocknet. Auf diese Masse werden dann noch zwei Schaumschichten mit der o.g. Rezeptur aufgegeben; die letzte wird mit einem Papiervlies (Kleenex Tuch) abgedeckt.

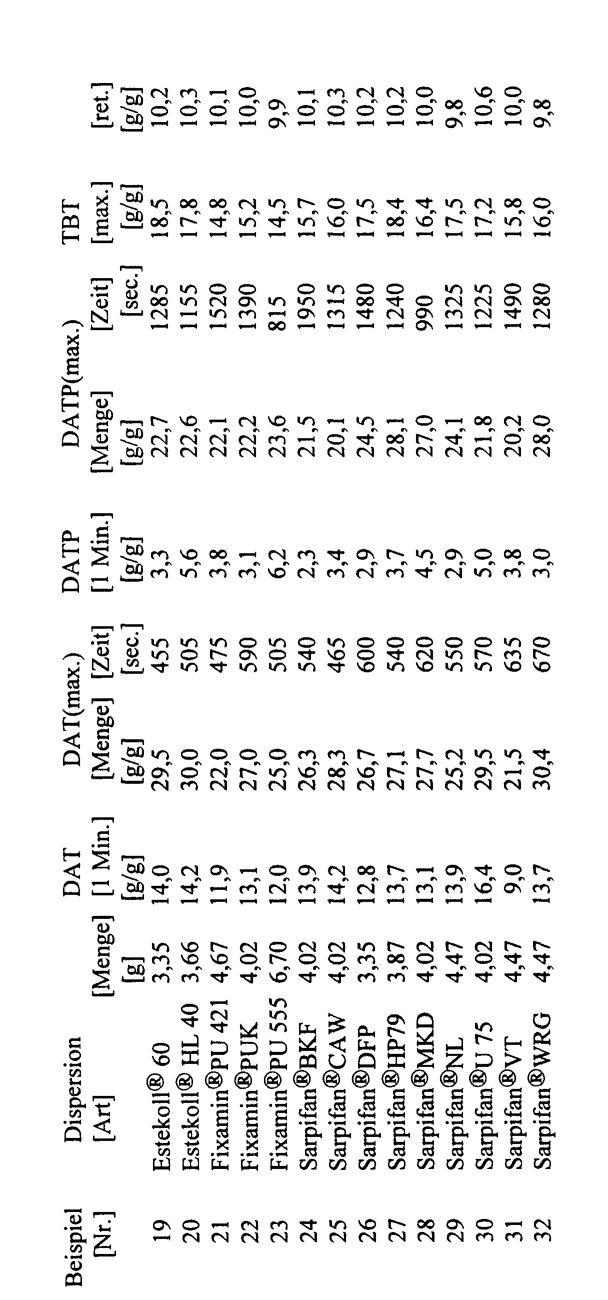

### Beispiele 33 - 37

50 g einer Latexdispersion, 3 g Mirox^{®} TA und 5 g deionisiertes Wasser werden zu einer Paste verrührt. Die Hälfte dieser Paste wird auf eine Fläche von 1000 cm² ausgestrichen und 1 Minute im Umlufttrockenschrank bei 160°C getrocknet. Dann wird die zweite Hälfte der Paste auf den entstandenen Film gestrichen und anschließend werden 20 g Favor^{®} SXM 100 aufgestreut. Dieser Körper wurde dann für 1 Minute auf 160°C erwärmt.
Ein Schaum aus 30 g der selben Latexdispersion, 3 g Plantaren^{®}2000 CS/UP und 10 g Wasser werden auf die bekannte Weise auf ein Schaumlitergewicht von ca. 250 g/l aufgeschäumt. Diese Masse wird gleichmäßig auf den hergestellten Körper verteilt und sofort anschließend für 1 Minute im IR Feld (10 kW/m²) erwärmt. Anschließend wird noch für 5 Minuten auf 160°C im Trockenschrank erhitzt.
Die Aufnahmegeschwindigkeit für syn. Urin wurde mittels DAT sowie DATP mit je 100 cm² großen Stücken bestimmt. Notwendig ist in diesem Falle, daß die Schaumschicht auf dem Flüssigkeitauslaß liegt, da die mit der Paste hergestellte Unterschicht flüssigkeitsundurchlässig ist.

| Beispiel | Dispersion | DAT | | DATP | | |
|---|---|---|---|---|---|---|
| [Nr.] | [Art] | [1'] | [5'] | [1'] | [5'] | [10'] |
| | | [g] | [g] | [g] | [g] | [g] |
| 33 | Bunatex^{®}SL 3510 | 23,2 | 46,1 | 9,8 | 30,5 | 41,6 |
| 34 | Bunatex^{®}SL 2810 | 20,2 | 42,2 | 6,2 | 19,7 | 31,8 |
| 35 | Sarpifan^{®}WRG | 16,8 | 42,5 | 2,2 | 2,2 | 2,2 |
| 36 | Acronal^{®} DS 2331 X | 16,8 | 38,6 | 7,6 | 21,8 | 31,6 |
| 37 | Sarpifan^{®}NL | 17,7 | 45,5 | 6,0 | 25,1 | 34,5 |

### Beispiele 38 - 42

Aus einem Schaum aus 0,3 g Plantaren^{®}2000 CS/UP, 6,0 g Bunatex^{®}SL 3510, 0,6 g VP-Non-Gel und 3,0 g deionisiertem Wasser wird ein Schaum bereitet (Schaumlitergewicht = 350 g/cm³) und auf eine Fläche von 300 cm² ausgestrichen. Der Schaum wird mit 3 g Favor^{®} SXM 100 bestreut, 1 Minute im IR Feld behandelt und anschließen eine bestimmte Zeit bei definierter Temperatur im Trockenschrank erwärmt. Anschließend wird eine Deckschicht aus dem gleichen Schaum aufgetragen und die Nachbehandlung wiederholt.

Je 50 cm² große Stücke dieser Körper werden im DAT auf ihre Aufnahmegeschwindigkeit hin untersucht. Dazu wird die Aufnahme nach einer, zwei, drei und vier Minuten notiert, sowie die Zeit, die notwendig war um 50 ml der syn. Urinlösung aufzunehmen.

| Beispiel | Temperatur/Zeit | DAT | | | | |
|---|---|---|---|---|---|---|
| [Nr.] | | [1'] | [2'] | [3'] | [4'] | [50 g erreicht] |
| | [°C]/[Min.] | [g] | [g] | [g] | [g] | [sec.] |
| 38 | 100/12 | 11,5 | 26,8 | 39,2 | 47,3 | 270 |
| 39 | 120/10 | 17,6 | 32,4 | 42,5 | 45,1 | 250 |
| 40 | 140/8 | 23,4 | 46,0 | 50,0 | 50,0 | 140 |
| 41 | 160/6 | 21,7 | 41,8 | 50,0 | 50,0 | 165 |
| 42 | 180/4 | 22,5 | 42,8 | 50,0 | 50,0 | 150 |

### Beispiele 43 - 46

Es werden Körper nach der gleiche Verfahrensweise hergestellt wie in den vorangegangenen Beispielen, jedoch werden den Schäumen unterschiedliche Mengen an VP-Non-Gel zugesetzt. Alle Schäume werden für 8 Minuten auf 140°C erhitzt.
Die Körper werden wie in den Beispielen 38 - 42 getestet.

| Beispiel | VP-Non-Gel | DAT | | | | |
|---|---|---|---|---|---|---|
| [Nr.] | | [1'] | [2'] | [3'] | [4'] | [50 g erreicht] |
| | [g/Schaumschicht] | [g] | [g] | [g] | [g] | [sec.] |
| 43 | 0 | 22,9 | 31,7 | 37,2 | 42,2 | 350 |
| 44 | 0,6 | 20,4 | 39,2 | 44,4 | 48,0 | 290 |
| 45 | 1,2 | 24,3 | 42,5 | 50,0 | 50,0 | 165 |
| 46 | 2,4 | 7,8 | 17,7 | 29,5 | 38,8 | 345 |

### Beispiele 47 - 50

Beispiel 45 wird wiederholt, jedoch werden in jede Schaumlage unterschiedliche Mengen an Titandioxid (Rutil - Typ) als Füllstoff eingearbeitet.

| Beispiel | Titandioxid | DAT | | | |
|---|---|---|---|---|---|
| [Nr.] | | [1'] | [2'] | [3'] | [50 g erreicht] |
| | [g] | [g] | [g] | [g] | [sec.] |
| 47 | 0,6 | 18,1 | 37,0 | 50,0 | 155 |
| 48 | 1,2 | 22,1 | 43,6 | 50,0 | 135 |
| 49 | 2,4 | 22,6 | 43,7 | 50,0 | 145 |
| 50 | 4,8 | 12,8 | 25,1 | 38,8 | 220 |

### Beispiele 51 - 54

Die vorherigen Beispiele werden wiederholt, jedoch mit dem Unterschied, daß nun 2,4 g von verschieden Füllstoffen in die Schäume eingearbeitet werden.

| Beispiel | Füllstoff | DAT | | | | |
|---|---|---|---|---|---|---|
| [Nr.] | [Art] | [1'] | [2'] | [3'] | [4'] | [50 g erreicht] |
| | | [g] | [g] | [g] | [g] | [sec.] |
| 51 | Bentonit | 16,0 | 32,3 | 50,0 | 50,0 | 170 |
| 52 | Kreide | 22,2 | 45,2 | 50,0 | 50,0 | 130 |
| 53 | Kaolin^{®}W | 1,5 | 2,9 | 4,3 | 7,0 | --- |
| 54 | Talkum | 21,5 | 40,9 | 50,0 | 50,0 | 145 |

### Beispiel 55,56

Versuch 52 wird mit 4,8 und 10 g Kreide als Füllstoff wiederholt. Die Probestücke zeigen im DAT nach 120 bzw. nach 105 Sekunden eine Aufnahme von 50 ml.

### Beispiele 57 - 59

Versuch 52 wird wiederholt, jedoch mit dem Unterschied, daß die Menge an Favor^{®} SXM 100 auf 300 g/m² gesteigert wird; außerdem wird die Konzentration der Kreide im Schaum weiter gesteigert. Es wurden Probestücke von 33,3 cm² im DATP getestet. Die Flüssigkeitsmenge die nach festgelegten Zeitspannen aufgenommen wurde, sowie die Zeit bis zur Aufnahme von 50 ml Testurinlösung waren die Prüfkriterien.

| Beispiel | Kreide | DATP | | | | | |
|---|---|---|---|---|---|---|---|
| [Nr.] | | [1'] | [2'] | [3'] | [4'] | [5'] | [50 ml erreicht] |
| | [g/Schaumsch.] | [g] | [g] | [g] | [g] | [g] | [sec.] |
| 57 | 9,6 | 16,5 | 28,3 | 35,5 | 41,0 | 44,9 | 350 |
| 58 | 14,5 | 23,6 | 38,7 | 47,4 | 50,0 | 50,0 | 290 |
| 59 | 19,3 | 15,5 | 27,1 | 35,5 | 41,2 | 45,5 | 165 |

### Beispiel 60

Auf ein 500 cm² großes Stück Polyethylenfolie (1,8 g) werden 20 g Estekoll^{®} HL 40 gestrichen; diese Schicht wird mit 15 g Favor^{®} SXM 100 gleichmäßig bestreut. Diese Masse wird 10 Minuten bei 100°C getrocknet. Anschließend wird ein Schaum aus 0,5 g Plantaren^{®}2000 CS/UP, 10 g Bunatex^{®}SL 3510, 2 g VP-Non-Gel und 5 g deionisiertem Wasser bereitet (Schaumlitergewicht ca. 250 g/l); in diesen Schaum werden noch 8,05 g Kreide eingearbeitet.
Die Masse aus dem Trockenschrank wird gleichmäßig mit diesem Schaum bestrichen, dieser Körper wird dann 1 Minute im IR - Feld und anschließend 10 Minuten bei 100°C im Trockenschrank nachbehandelt.
Ein 33,3 cm² großes Flächenstück dieses Körpers zeigt im DAT nach 9 Minuten eine Aufnahme von 50 ml syn. Urinlösung. Die aufgebrachte Polyethylenfolie ist flüssigkeitsundurchlässig, d. h. der DAT - Test wird immer so durchgeführt, daß die Schaumschicht mit der Flüssigkeit in Kontakt steht.

### Beispiel 61

Es wird wie in Beispiel 60 verfahren, jedoch wird diesmal als Unterschicht eine Mischung aus 10 g Estekoll^{®} HL 40, 10 g Wasser und 2,5 g Mirox^{®} TA auf die Polyethylenfolie aufgetragen. Außerdem wurde eine weitere Schaumschicht mit gleicher Rezeptur aufgetragen.
Ein 33,3 cm² großes Flächenstück dieses Körpers zeigt im DAT nach 4,25 Minuten eine Aufnahme von 50 ml syn. Urinlösung.

### Beispiel 62,63

Beispiel 58 wurde wiederholt, jedoch wird statt der Non-Gel-Paste Neogel^{®} V 70 ZB bzw. Neogel^{®} V 77 ZB verwendet. Im Falle des Neogel^{®} V70 ZB zeigte ein Prüfkörper nach 230 Sekunden eine Aufnahme von 50 ml syn. Urin (DAT) im Falle des Neogel® V 77 ZB nach 200 Sekunden.

### Beispiele 64 - 71

1,7 g Plantaren^{®}2000 CS/UP, 5,21 g Neogel^{®}V 70 ZB, 13,02 g deionisiertes Wasser werden mit einer Polymerdispersion gemischt und auf ca. 300 g/l aufgeschäumt. Anschließend wird ggf. ein Füllstoff in den Schaum gemischt. Dann wird dieser auf eine Fläche von 1302 cm² ausgestichen und mit 39,06 g Favor^{®}SXM 100 bestreut. Die Masse wird anschließend 4 Minuten bei 180°C im Trockenschrank erhitzt, mit einer weiteren, gleichen Schaumschicht bestrichen und abermals 4 Minuten bei 180 ° C getrocknet. Vom entstandenen Körper werden 10 x 30 cm große Stücke ausgeschnitten; mit diesen wird der Oberflächentest durchgeführt.

| Bsp. | Dispersion/Menge | Füllstoff/Menge |
|---|---|---|
| 64 | Bunatex^{®}SL 3510/26,04 g | -/- |
| 65 | Sarpifan^{®}MKD/35,41 g | -/- |
| 66 | Bunatex^{®}SL 3510/26,04 g | Kreide/82,39 g |
| 67 | Bunatex^{®}SL 3510/26,04 g | Calcicoll^{®}W 12/82,39 g |
| 68 | Sarpifan^{®}MKD/35,41 g | Kreide/82,39 g |
| 69 | Bunatex^{®}SL 3510/26,04 g | Kreide/41,2 g |
| 70 | Bunatex^{®}SL 3510/26,04 g | Calcicoll^{®}W 12/41,2 g |
| 71 | Sarpifan^{®}MKD/35,41 g | Kreide/41,2 g |

| Bsp | | -1- | | | -2- | | | -3- | | | -4- | | | -5- | |
|---|---|---|---|---|---|---|---|---|---|---|---|---|---|---|---|
| | OA | ZA | AV | OA | ZA | AV | OA | ZA | AV | OA | ZA | AV | OA | ZA | AV |
| | [ml] | [s] | [ml] | [ml] | [s] | [ml] | [ml] | [s] | [ml] | [ml] | [s] | [ml] | [ml] | [s] | [ml] |
| 64 | 0,5 | 0 | 43 | 0 | 20 | 21 | 0 | 25 | 21 | 0 | 25 | 32 | 0 | 20 | 55 |
| 65 | 0 | 20 | 10 | 0 | 25 | 2 | --- | --- | --- | --- | --- | --- | --- | --- | --- |
| 66 | 0 | 15 | 23 | 0 | 45 | 5 | 0 | 40 | 15 | 0 | 40 | 29 | 0 | 30 | 63 |
| 67 | 0 | 40 | 3 | 0 | --- | 0 | 0 | --- | 0 | 0 | 30 | 0,5 | --- | --- | --- |
| 68 | 14 | --- | 14 | 0 | --- | 0 | 0 | --- | 0 | 0 | 300 | 5 | --- | --- | --- |
| 69 | 1 | 0 | 32 | 0 | 20 | 15 | 0 | 35 | 20 | 0 | 40 | 35 | 0 | 30 | 52 |
| 70 | 0,5 | 0 | 33 | 0 | 30 | 18 | 0 | 30 | 25 | 0 | 40 | 32 | 0 | 35 | 55 |
| 71 | 0 | 15 | 14 | 0 | --- | 0 | 0 | --- | 0 | 0 | 60 | 25 | --- | --- | --- |

### Beispiele 72 - 75

0,48 g Plantaren^{®}2000 CS/UP, 0,17 g Magnesiumchlorid (x 6 H₂O), 2,38 g deionisiertes Wasser werden mit 6,48 g Sarpifan^{®}MKD, 0,33g Fixapet^{®}VNF und Kreide auf ca. 300 g/l (berechnet ohne Kreide) aufgeschäumt. Dann wird dieser Schaum auf eine Fläche von 14 x 34 cm ausgestrichen und auf einer Fläche von 10 x 30 cm mit 4,5 g Favor^{®}SXM 100 bestreut. Die Masse wird anschließend 3 Minuten bei 180°C im Trokkenschrank erhitzt, mit einer weiteren, gleichen Schaumschicht bestrichen noch mal mit der gleichen Menge an Favor^{®}SXM 100 bestreut und abermals 3 Minuten bei 180 ° C getrocknet. Darauf wird eine Deckschicht aus dem gleichen Schaummaterial aufgestrichen und ein drittes Mal bei 180 ° C im Trockenschrank getrocknet (vgl. Abb. 1). Anschließend wurden die Proben kalandert.

| Bsp | Kreide | | -1- | | | -2- | | | -3- | | | -4- | |
|---|---|---|---|---|---|---|---|---|---|---|---|---|---|
| | | OA | ZA | AV | OA | ZA | AV | OA | ZA | AV | OA | ZA | AV |
| | [g] | [ml] | [s] | [ml] | [ml] | [s] | [ml] | [ml] | [s] | [ml] | [ml] | [s] | [ml] |
| 72 | 5,71 | 0 | -- | 0 | 0 | 30 | 0,1 | 0 | 30 | 18 | 0 | 20 | 62 |
| 73 | 3,81 | 0 | -- | 0 | 7,0 | 15 | 11 | 0 | 30 | 19 | 0 | 20 | 60 |
| 74 | 1,90 | 0 | 40 | 0,5 | 24 | 0 | 25 | 13 | 5 | 18 | 23 | 10 | 58 |
| 75 | 0,00 | 0 | 20 | 11 | 28 | 5 | 30 | 45 | 5 | 47 | 52 | 5 | 54 |

### Beispiele 76 - 93

0,50 g Plantaren^{®}2000 CS/UP, 0,09 g Magnesiumchlorid (x 6 H₂O), 2,4 g deionisiertes Wasser werden mit 3,2 g Sarpifan^{®}MKD, 0,17g Fixapet^{®}VNF und 5,44 g Kreide sowie unter Zusatz einer bestimmten Menge einer zweiten Polymerdispersion auf ca. 300 g/l (berechnet ohne Kreide) aufgeschäumt. Dann wird dieser Schaum auf eine Fläche von 14 x 34 cm ausgestrichen und auf einer Fläche von 10 x 30 cm mit 4,5 g Favor^{®}SXM 100 bestreut. Die Masse wird anschließend 3 Minuten bei 180°C im Trokkenschrank erhitzt, mit einer weiteren, gleichen Schaumschicht bestrichen, noch mal mit der gleichen Menge an Favor^{®}SXM 100 bestreut und abermals 3 Minuten bei 180 ° C getrocknet. Darauf wird eine Deckschicht aus dem gleichen Schaummaterial aufgestrichen und ein drittes Mal bei 180 ° C im Trockenschrank getrocknet. Anschließend wurden die Proben (soweit nicht anders vermerkt) kalandert.

| Beispiel Nr. Bemerkungen | Polymerdispersion | Einwaage | |
|---|---|---|---|
| 76 | Fixamin PUK | 3,20 g | |
| 77 | Sarpifan^{®}BKF | 3,20 g | instabil |
| 78 | Sarpifan^{®}CAW | 3,20 g | |
| 79 | Sarpifan^{®}DFP | 2,67 g | |
| 80 | Sarpifan^{®}HP79 | 3,08 g | |
| 81 | Sarpifan^{®}NL | 3,56 g | |
| 82 | Sarpifan^{®}U 75 | 3,20 g | |
| 83 | Sarpifan^{®}VBA | 3,20 g | |
| 84 | Sarpifan^{®}VT | 3,56 g | |
| 85 | Sarpifan^{®}WRG | 3,56 g | |
| 86 | Estekoll^{®} 60 | 2,67 g | |
| 87 | Estekoll^{®} HL 40 | 2,91 g | |
| 88 | Bunatex^{®}SL 2800 | 2,39 g | |
| 89 | Bunatex^{®}SL 3510 | 2,35 g | |
| 90 | Acronal^{®} DS 2331 X | 3,59 g | |
| 91 | Vinipas^{®}LL 778/5 | 3,20 g | |
| 92 | Lipolan^{®}VD 9910 | 3,20 g | nicht kalandert |
| 93 | Litex^{®}AP 4120 | 3,20 g | |

| Bsp | | -1- | | | -2- | | | -3- | | | -4- | | | -5- | |
|---|---|---|---|---|---|---|---|---|---|---|---|---|---|---|---|
| | OA | ZA | AV | OA | ZA | AV | OA | ZA | AV | OA | ZA | AV | OA | ZA | AV |
| | [ml] | [s] | [ml] | [ml] | [s] | [ml] | [ml] | [s] | [ml] | [ml] | [s] | [ml] | [ml] | [s] | [ml] |
| 76 | 0 | 100 | 2 | 3,0 | 5 | 5 | 0 | 45 | 16 | 0 | 30 | 60 | 0 | 30 | 77 |
| 77 | --- | --- | --- | --- | --- | --- | --- | --- | --- | --- | --- | --- | --- | --- | --- |
| 78 | 0 | -- | 0 | 0 | -- | 0 | 0 | 30 | 15 | 0 | 20 | 60 | 0 | 20 | 78 |
| 79 | 0 | 45 | 3 | 15 | 10 | 18 | 0 | 30 | 18 | 0 | 30 | 52 | 0 | 30 | 76 |
| 80 | 0 | -- | 0 | 0 | 15 | 8 | 0 | 25 | 18 | 0 | 30 | 52 | 0 | 30 | 75 |
| 81 | 0 | -- | 0 | 0 | -- | 0 | 0 | 120 | 4 | 0 | 45 | 47 | 0 | 45 | 76 |
| 82 | 0 | -- | 0 | --^{*} | --- | --- | --- | --- | --- | --- | --- | --- | --- | --- | --- |
| 83 | 0 | -- | 0 | 0 | -- | 0 | 0 | 70 | 9 | 0 | 30 | 55 | 0 | 30 | 80 |
| 84 | 10 | 15 | 23 | 34 | 0 | 37 | 28 | 0 | 35 | 20 | 0 | 45 | 16 | 15 | 67 |
| 85 | 0 | -- | 0 | 0 | -- | 0 | 0 | 60 | 10 | 0 | 30 | 58 | 0 | 30 | 77 |
| 86 | 0 | -- | 0 | 0 | 40 | 1 | 0 | 45 | 15 | 0 | 30 | 56 | 0 | 20 | --- |
| 87 | 0 | 35 | 1 | 14 | 5 | 17 | 0 | 30 | 20 | 0 | 20 | 60 | --- | --- | --- |
| 88 | 10 | -- | 10 | 3 | 15 | 7 | 0 | 30 | 13 | 0 | 20 | --- | --- | --- | --- |
| 89 | 14 | 0 | 30 | 0 | 30 | 4 | 0 | 30 | 10 | 0 | 30 | --- | --- | --- | --- |
| 90 | 0 | 30 | 18 | 6 | 10 | 12 | 0 | 30 | 14 | --- | --- | --- | --- | --- | --- |
| 91 | 0 | 45 | 6 | 0 | 20 | 8 | 0 | 45 | 20 | --- | --- | --- | --- | --- | --- |
| 92 | 0 | 10 | 65 | 0 | 20 | 36 | 0 | 30 | 32 | --- | --- | --- | --- | --- | --- |
| 93 | 0 | 40 | 2 | 0 | 35 | 1 | 0 | 35 | 18 | 0 | 25 | 56 | --- | --- | --- |

Die Versuche wurden vor der 5. Aufgabe abgebrochen, wenn der Versuchskörper zwischenzeitlich während des Versuchs gerissen war.

### Beispiele 94 - 96

Es wird aus 8 g Plantaren^{R}2000 CS/UP, 8 g Stokal^{R}SR, x g Sarpifan^{R}VBA, 100 g H₂O und y g Sarpifan^{R}MKD ein Schaum mit einem Litergewicht von 300 g/l bereitet. Dieser wird auf einer Fläche von 1 m² ausgestrichen, mit 200 g Favor^{R}SXM 100 gleichmäßig bestreut und anschließend 5 Minuten bei 160 °C getrocknet. Auf dieses Gebilde wird eine gleiche Schaumschicht aufgetragen, mit 100 g Favor^{R}SXM 100 gleichmäßig bestreut, und anschließend wird abermals bei 160 °C 5 Minuten getrocknet. Anschließend wird noch zweimal eine gleiche Schaumschicht aufgetragen und wie zuvor getrocknet
Die Aufnahmegeschwindigkeit wird im DAT-Test nach einer Minute bestimmt.

| Beispiel Nr. | Sarpifan^{R}VBA | Sarpifan^{R}MKD | DAT (1 Minute) |
|---|---|---|---|
| | [g/Schaumschicht] | [g/Schaumschicht] | [g/g] |
| 94 | 114,1 | 20,1 | 9,3 |
| 95 | 94,0 | 40,2 | 13,1 |
| 96 | 67,1 | 67,1 | 12,3 |

### Beispiel 97

12,5 g Plextol MV 604, 12,5 g Plextol DV 440 (Acrylatdispersionen der Röhm GmbH) 1 g Fixapret VNF, 0,1 g Magnesiumchlorid, 2 g Wasser und 1 g Glucosid 81 S (Alkylpolyglucosid der Hüls AG, Marl) werden vermischt.
Die Mischung wird mit einem Handrührgerät auf 40 g/l Schaumlitergewicht aufgeschlagen. Die Hälfte des so entstandenen Schaums wird auf die Flächen von 40 * 50 cm ausgestrichen und mit 40 g Favor SXM 100 bestreut.
Das so entstandene Gebilde wird mit der zweiten Hälfte des Schaums abgedeckt. Anschließend wird für 4 Minuten bei 200°C im Umlufttrockenschrank getrocknet.
Die entstandene Fläche weist eine hohe Flexibilität auf.
TBT (ret.):6,61(0,9%NaCl)/m².

### Beispiel 98

Beispiel 97 wird wiederholt, jedoch wird zusätzlich vor dem Schäumen 2 g des Alkylglucosids und 7,5 g Wasser zugesetzt. Das Schaumlitergewicht des mit dieser Mischung bereiteten Schaums beträgt 30 g/l.
Das entstandene Flächengebilde weist eine gröbere Porenstruktur auf als das in Beispiel 97 hergestellte, die Aufnahmekapazität beträgt ebenfalls 6,61/m².

## Patentansprüche

1. Schichtförmiger, aus einer oder mehreren Kunststoffschaumschichten und/oder Latexschaumschichten und partikelförmigen, superabsorbierenden Polymerisaten bestehender Körper zur Absorption von Wasser und wässrigen Flüssigkeiten, dadurch gekennzeichnet, daß direkt auf, zwischen oder unter den geschäumten Kunststoffschichten und/oder geschäumten Latexschichten das superabsorbierende Polymerisat in mengenmäßig und/oder örtlich vorgegebener und fixierter flächenmäßiger Anordnung enthalten ist und das Mengenverhältnis von geschäumter Kunststoff-und/oder geschäumter Latexschicht zum superabsorbierenden Polymerisat 1:500 bis 50:1 beträgt, wobei der Schichtaufbau durch Aufbringen des partikelförmigen, superabsorbierenden Polymerisates in bestimmter mengenmäßiger Verteilung auf die Oberfläche eines zuvor hergestellten, flächenförmig verteilten Kunststoff- und/oder Latexschaumes erfolgt und die Kunststoff- und/oder Latexschaumschicht durch Wärmebehandlung, oder durch Behandlung im UV-Feld vulkanisiert und das superabsorbierende Polymerisat fixiert worden ist, wobei die Erzeugung einer flächenförmig verteilten Kunststoff- und/oder Latexschaumschicht und das Aufbringen des partikelförmigen, superabsorbierenden Polymerisates gegebenenfalls in beliebiger Reihenfolge wiederholt worden ist, und abschließend durch eine Wärmebehandlung unter Vernetzung der geschäumten Schichten erfolgt ist.

2. Schichtförmiger, aus einer oder mehreren Kunststoffschaumschichten und/oder Latexschaumschichten und partikelförmigen, superabsorbierenden Polymerisaten bestehender Körper nach Anspruch 1, dadurch gekennzeichnet, daß das Mengenverhältnis von geschäumter Kunststoff- und/oder geschäumter Latexschicht zum superabsorbierenden Polymerisat 1:50 bis 25:1 beträgt.

3. Schichtförmiger, aus einer oder mehreren Kunststoffschaumschichten und/oder Latexschaumschichten und partikelförmigen, superabsorbierenden Polymerisaten bestehender Körper nach Anspruch 1 oder 2, dadurch gekennzeichnet, daß das Mengenverhältnis von geschäumter Kunststoff- und/oder geschäumter Latexschicht zum superabsorbierenden Polymerisat 1:5 bis 10:1 beträgt.

4. Schichtförmiger, aus einer oder mehreren Kunststoffschaumschichten und/oder Latexschaumschichten und partikelförmigen, superabsorbierenden Polymerisaten bestehender Körper nach einem der Ansprüche 1 bis 3, dadurch gekennzeichnet, daß er mit einer 0,9 %-iger NaCl-Lösung
eine Retention von mindestens 0,1 Liter/m² Oberfläche,
eine maximale Aufnahme von mindestens 0,1 Liter/m² Oberfläche,
und eine Absorption unter Belastung (AUL) von mindestens 2 g/g bei 2,1 kPa aufweist.

5. Schichtförmiger, aus einer oder mehreren Kunststoffschaumschichten und/oder Latexschaumschichten und partikelförmigen, superabsorbierenden Polymerisaten bestehender Körper nach einem der Ansprüche 1 bis 4, dadurch gekennzeichnet, daß die einzelne Kunststoff- und/oder Latexschaumschicht und/oder das superabsorbierende Polymerisat zusätzlich wenigstens ein Füllstoffmaterial bis zu einem Füllungsgrad von höchstens 1000 Gew.-%, bevorzugt bis zu einem Füllungsgrad von höchstens 400 Gew.-% und besonders bevorzugt von höchstens 200 Gew.%, bezogen auf die Menge des Kunststoff- und/oder Latexschaums, enthält.

6. Schichtförmiger, aus einer oder mehreren Kunststoffschaumschichten und/oder Latexschaumschichten und partikelförmigen, superabsorbierenden Polymerisaten bestehender Körper nach einem der Ansprüche 1 bis 5, dadurch gekennzeichnet, daß die Kunststoffschaumschicht und/oder Latexschaumschicht und/oder das superabsorbierende Polymerisat als Füllstoffmaterial Kreiden, Bentonite, Kieselgele, Kieselsäuren, Aktivkohle, Pigmente, bevorzugt Titandioxid und/oder Eisenoxid, und/oder natürliche und/oder synthetische Fasermaterialien, bevorzugt Viskose- und Baumwollfasern und -gewebe und/oder Polyester- und Polyamidfasern, Mischungen verschiedener Fasern oder Gewebe und/oder feingemahlene Kunststoffe enthält.

7. Schichtförmiger, aus einer oder mehreren Kunststoffschaumschichten und/oder Latexschaumschichten und partikelförmigen, superabsorbierenden Polymerisaten bestehender Körper nach einem der Ansprüche 1 bis 6 dadurch gekennzeichnet, daß die Oberfläche des Körpers teilweise, vorzugsweise einseitig, eine für wäßrige Flüssigkeiten und Wasser undurchlässige Schicht, eine Kunststoffolie und/oder ein Gewebe, enthält und/oder teilweise, vorzugsweise einseitig, eine Schicht aus Zellstoff, Vlies und/oder Papier und/oder ein textiles Vlies oder Gewebe enthält.

8. Verfahren zu Herstellung eines schichtförmig, aus einer oder mehreren Kunststoffschaumschichten und/oder Latexschaumschichten und partikelförmigen, superabsorbierenden Polymerisaten bestehendem Körper zur Absorption von Wasser und wäßrigen Flüssigkeiten, dadurch gekennzeichnet, daß mindestens
a) ein Kunststoff- und/oder Latexschaum mit einem Litergewicht von 50 bis 1000 g/l erzeugt und
der Schaum flächenförmig in einer Schichtdicke von 1 µm bis 100.000 µm, vorzugsweise von 10 µm bis 10.000 µm, besonders bevorzugt von 200 µm bis 5.000 µm, verteilt wird,
b) das superabsorbierende, partikelförmige Polymerisat in einem Mengenverhältnis von geschäumter Kunststoff- und/oder geschäumter Latexschicht zum superabsorbierenden Polymerisat von 1 : 500 bis 50 : 1, vorzugsweise 1 : 50 bis 25 : 1, besonders bevorzugt 1 : 5 bis 10 : 1, gegebenenfalls unter Verwendung mindestens einer Schablone, einer Lochscheibe und/oder eines Siebes in bestimmter mengen- und flächenmäßiger Verteilung auf den flächenförmig verteilten Kunststoff- und/oder Latexschaum aufgebracht, die Kunstoff- und/oder Latexschaumschicht durch Wärmebehandlung, oder durch Behandlung im UV-Feld vulkanisiert und das superabsorbierende Polymerisat fixiert wird, wobei gegebenenfalls der (die) Verfahrensschritt(e) a) und/oder b) in beliebiger Reihenfolge wiederholt wird/werden, und abschließend eine Wärmebehandlung unter Vernetzung der geschäumten Schicht(en) erfolgt.

9. Verfahren zur Herstellung eines schichtförmig, aus einer oder mehreren Kunststoffschaumschichten und/oder Latexschaumschichten und partikelförmigen, superabsorbierenden Polymerisaten bestehendem Körper nach Anspruch 8, dadurch gekennzeichnet, daß ein superabsorbierendes Polymerisat mit einer Korngrößenverteilung von 1 µm bis 20.000 µm verwendet wird.

10. Verfahren zur Herstellung eines schichtförmig, aus einer oder mehreren Kunststoffschaumschichten und/oder Latexschaumschichten und partikelförmigen, superabsorbierenden Polymerisaten bestehendem Körper nach Anspruch 8 oder 9, dadurch gekennzeichnet, daß die Herstellung unter Verwendung von Hilfsmaterialien, wie Metall- oder Glasflächen, Kunststofffolien und/oder Siliconpapier erfolgt, von denen der schichtförmige Körper nach seiner Herstellung abgetrennt wird, oder daß die Herstellung mit Hilfe mindestens einer Zellstoff-, Vlies- oder Papierschicht und/oder einem textilen Vlies und/oder Gewebe als Mittelschicht, Grundschicht oder abschließender Deckschicht erfolgt.

11. Verfahren zur Herstellung eines schichtförmig, aus einer oder mehreren Kunststoffschaumschichten und/oder Latexschaumschichten und partikelförmigen, superabsorbierenden Polymerisaten bestehendem Körper nach einem der Ansprüche 8 bis 10, dadurch gekennzeichnet, daß die Menge, Korngröße und Verteilung des partikelförmigen, superabsorbierenden Polymerisats auf den einzelnen geschäumten Kunststoffschichten und/oder geschäumten Latexschichten verschieden ist.

12. Verfahren zur Herstellung eines schichtförmig, aus einer oder mehreren Kunststoffschaumschichten und/oder Latexschaumschichten und partikelförmigen, superabsorbierenden Polymerisaten bestehendem Körper nach einem der Ansprüche 8 bis 11, dadurch gekennzeichnet, daß vor, während oder nach der Herstellung der einzelnen Kunststoff- und/oder Latexschaumschicht als Füllstoffe Bentonite, Kieselgele, Kieselsäuren, Aktivkohle, anorganische Pigmente, gemahlene Kunststoffe, vorzugsweise Kreide und/oder natürliche und/oder synthetische Fasern mit einem Füllungsgrad von 0 - 1000 Gew.%, bezogen auf den Kunststoffschaum und/oder Latexschaum, vorzugsweise bis maximal 400 Gew.%, besonders bevorzugt bis maximal 200 Gew.% in den Schaum gegebenenfalls zusammen mit einem oder mehreren Verdickungsmitteln, Vernetzern und Stabilisatoren eingebracht werden.

13. Verfahren zur Herstellung eines schichtförmig, aus einer oder mehreren Kunststoffschaumschichten und/oder Latexschaumschichten und partikelförmigen, superabsorbierenden Polymerisaten bestehendem Körper nach einem der Ansprüche 8 bis 12, dadurch gekennzeichnet, daß die Schaumbildung durch Mischen zweier oder mehrerer Kunststoffe und/oder Latices und gegebenenfalls unter Zusatz von Hilfsmitteln, wie Schäumungsmittel, Blähmittel, Schaumstabilisatoren, Vernetzern und Vulkanisationsmitteln erfolgt.

14. Verfahren zur Herstellung eines schichtförmig, aus einer oder mehreren Kunststoffschaumschichten und/oder Latexschaumschichten und partikelförmigen, superabsorbierenden Polymerisaten bestehendem Körper nach einem der Ansprüche 8 bis 13 dadurch gekennzeichnet, daß der schichtförmige Körper abschließend mit einem Kalander und/oder einer Prägewalze bearbeitet wird.

15. Verwendung des schichtförmigen Körpers nach einem der Ansprüche 1 bis 7 in Hygieneartikeln und im sanitären Bereich zur Aufnahme von Wasser und Körperflüssigkeiten.

16. Verwendung des schichtförmigen Körpers nach Anspruch 15 als Absorber-Komponente in Babywindeln und in Inkontinenzartikeln.

17. Verwendung des schichtförmigen Körpers nach einem der Ansprüche 1 bis 7, direkt oder als Komponente in natürlichen und/oder künstlichen Böden zur Pflanzenzucht oder zum Transport und zur Lagerung von Pflanzen oder Pflanzenteilen.

18. Verwendung des schichtförmigen Körpers nach einem der Ansprüche 1 bis 7 als Isolationsmaterial für Rohre und Leitungen, vorzugsweise für elektrische und lichtleitende Kabel.

19. Verwendung des schichtförmigen Körpers nach einem der Ansprüche 1 bis 7 als Isolationsmaterial für Baukonstruktionen, vorzugsweise in Außenmauern.

20. Verwendung des schichtförmigen Körpers nach einem der Ansprüche 1 bis 7, direkt oder als flüssigkeitsaufnehmende und/oder flüssigkeitsspeichernde Komponente in Verpackungmaterialien.

21. Verwendung des schichtförmigen Körpers nach einem der Ansprüche 1 bis 7 als Teil in Bekleidungsstücken.

## Claims

1. A layered element for absorbing water and aqueous liquids, consisting of one or more plastic foam layers and/or latex foam layers and particulate superabsorbent polymers, characterized in that the superabsorbent polymer is contained directly on top of, between or underneath the foamed plastic layers and/or foamed latex layers in a quantitatively and/or locally predetermined and fixed sheet-like arrangement, and the quantity ratio of foamed plastic layer and/or foamed latex layer to said superabsorbent polymer is from 1:500 to 50:1, whereby the layer structure has been accomplished by applying the particulate superabsorbent polymer with a specific distribution with respect to quantity onto the surface of a previously produced plastic foam and/or latex foam spread in a sheet-like fashion, and the plastic and/or latex foam layer has been cured by heat treatment or by treatment in a UV field and the superabsorbent has been fixed, whereby producing a plastic and/or latex foam layer spread in a sheet-like fashion and applying the particulate superabsorbent polymer optionally has been repeated in any order and finally, a heat treatment with crosslinking of the foamed layers has been effected.

2. The layered element according to claim 1, consisting of one or more plastic foam layers and/or latex foam layers and particulate superabsorbent polymers, characterized in that the quantity ratio of foamed plastic layer and/or foamed latex layer to said superabsorbent polymer is from 1:50 to 25:1.

3. The layered element according to claim 1 or 2, consisting of one or more plastic foam layers and/or latex foam layers and particulate superabsorbent polymers, characterized in that the quantity ratio of foamed plastic layer and/or foamed latex layer to said superabsorbent polymer is from 1:5 to 10:1.

4. The layered element according to any of claims 1 to 3, consisting of one or more plastic foam layers and/or latex foam layers and particulate superabsorbent polymers, characterized in that when using a 0.9% NaCl solution, the layered element has
- a retention of at least 0.1 liters/m² surface area,
- a maximum absorption of at least 0.1 liters/m² surface area, and
- an absorption under load (AUL) of at least 2 g/g at 2.1 kPa.

5. The layered element according to any of claims 1 to 4, consisting of one or more plastic foam layers and/or latex foam layers and particulate superabsorbent polymers, characterized in that a single plastic and/or latex foam layer and/or the superabsorbent polymer additionally include at least one filler material up to a filling level of 1000 wt.-% at maximum, preferably up to a filling level of 400 wt.-% at maximum, and more preferably 200 wt.-% at maximum, relative to the amount of plastic and/or latex foam.

6. The layered element according to any of claims 1 to 5, consisting of one or more plastic foam layers and/or latex foam layers and particulate superabsorbent polymers, characterized in that the plastic foam layer and/or latex foam layer and/or the superabsorbent polymer include chalks, bentonites, silica gels, silicic acids, active charcoal, pigments, preferably titanium dioxide and/or iron oxide, and/or natural and/or synthetic fiber materials, preferably viscose and cotton fibers and fabrics, and/or polyester and polyamide fibers, mixtures of various fibers or fabrics, and/or finely ground plastics.

7. The layered element according to any of claims 1 to 6, consisting of one or more plastic foam layers and/or latex foam layers and particulate superabsorbent polymers, characterized in that part of the surface of the element, preferably one side, includes a layer impermeable for aqueous liquids and water, a plastic film and/or fabric, and/or that part of the surface of the element, preferably one side, includes a layer of cellulose, fleece and/or paper and/or a textile fleece or fabric.

8. A process for producing a layered element for absorbing water and aqueous liquids, consisting of one or more plastic foam layers and/or latex foam layers and particulate superabsorbent polymers, characterized in that at least
a) one plastic and/or latex foam having a weight per liter of from 50 to 1000 g/l is produced, and the foam is spread in a sheet-like fashion at a layer thickness of from 1 µm to 100,000 µm, preferably from 10 µm to 10,000 µm, and more preferably, from 200 µm to 5000 µm,
b) the superabsorbent particulate polymer at a quantity ratio of foamed plastic layer and/or foamed latex layer to superabsorbent polymer of from 1:500 to 50:1, preferably from 1:50 to 25:1, and more preferably, from 1:5 to 10:1, is applied at a specific distribution with respect to quantity and area onto the plastic and/or latex foam spread in sheet-like fashion, optionally using at least one template, one perforated disk and/or one screen, the plastic and/or latex foam layer is cured by heat treatment or by treatment in a UV field and the superabsorbent is fixed, the processing step(s) a) and/or b) optionally being repeated in any order and finally, a heat treatment with crosslinking of the foamed layer(s) is effected.

9. The process according to claim 8 for producing a layered element consisting of one or more plastic foam layers and/or latex foam layers and particulate superabsorbent polymers, characterized in that a superabsorbent polymer having a grain size distribution of from 1 µm to 20,000 µm is used.

10. The process according to claim 8 or 9 for producing a layered element consisting of one or more plastic foam layers and/or latex foam layers and particulate superabsorbent polymers, characterized in that the production is performed using auxiliary materials such as metal or glass surfaces, plastic films and/or silicone paper from which the layered element is removed subsequent to its production, or that the production is performed using at least one cellulose, fleece or paper layer and/or a textile fleece and/or fabric as central layer, base layer or final cover layer.

11. The process according to any of claims 8 to 10 for producing a layered element consisting of one or more plastic foam layers and/or latex foam layers and particulate superabsorbent polymers, characterized in that the amount, grain size and distribution of the particulate, superabsorbent polymer on the individual foamed plastic layers and/or foamed latex layers are subject to variation.

12. The process according to any of claims 8 to 11 for producing a layered element consisting of one or more plastic foam layers and/or latex foam layers and particulate superabsorbent polymers, characterized in that bentonites, silica gels, silicic acids, active charcoal, inorganic pigments, ground plastics, preferably chalk and/or natural and/or synthetic fibers, optionally together with one or more thickening agents, crosslinkers and stabilizers, are incorporated as fillers in the foam prior to, during or subsequent to the production of the individual plastic and/or latex foam layers at filling levels of from 0 to 1000 wt.-%, relative to the plastic foam and/or latex foam, preferably up to 400 wt.-% at maximum, and more preferably, up to 200 wt.-% at maximum.

13. The process according to any of claims 8 to 12 for producing a layered element consisting of one or more plastic foam layers and/or latex foam layers and particulate superabsorbent polymers, characterized in that foam forming is effected by mixing two or more plastic materials and/or latices, optionally with addition of auxiliary agents such as foaming agents, expanding agents, foam stabilizers, crosslinkers, and curing agents.

14. The process according to any of claims 8 to 13 for producing a layered element consisting of one or more plastic foam layers and/or latex foam layers and particulate superabsorbent polymers, characterized in that the layered element is subjected to a final treatment using a calender and/or an embossing roller.

15. Use of the layered element according to any of claims 1 to 7 in hygiene articles and in the sanitary field for absorbing water and body fluids.

16. The use of the layered element according to claim 15 as an absorber component in diapers for babies and in incontinence articles.

17. Use of the layered element according to any of claims 1 to 7 in a direct fashion or as a component in natural and/or artificial soils for plant breeding or in the transportation and storage of plants or plant. parts.

18. Use of the layered element according to any of claims 1 to 7 as insulating material for tubes and pipings, preferably for electric and light-conducting cables.

19. Use of the layered element according to any of claims 1 to 7 as insulating material for building constructions, preferably in external walls.

20. Use of the layered element according to any of claims 1 to 7 in a direct fashion or as liquid-absorbing and/or liquid-storing component in packaging materials.

21. Use of the layered element according to any of claims 1 to 7 as part in clothing articles.

## Revendications

1. Corps constitué de couches destiné à l'absorption de l'eau et des liquides aqueux composé d'une ou plusieurs couches de mousse synthétique et/ou de couches de mousse de latex, et de polymères superabsorbants en forme de particules, caractérisé en ce que le polymère superabsorbant est contenu directement sur, entre, ou sous les couches de mousse synthétique et/ou les couches de mousse en latex dans un agencement de surface établi et déterminé quantitativement et/ou localement et en ce que le rapport quantitatif de la couche de mousse synthétique et/ou de la couche de mousse de latex au polymère superabsorbant est de 1 : 500 à 50 : 1, dans lequel la constitution de la couche s'effectue par application du polymère superabsorbant formé de particules en une répartition déterminée quantitativement sur la surface d'une mousse synthétique et/ou d'une mousse en latex fabriquée préalablement répartie de manière planéiforme, dans lequel la couche de mousse synthétique et/ou la mousse de latex ont été vulcanisées par traitement thermique ou par traitement aux rayons UV et le polymère superabsorbant y a été fixé, et dans lequel les opérations de fabrication d'une mousse synthétique et/ou d'une mousse en latex répartie en surface et d'application d'un polymère superabsorbant formé de particules ont été répétées éventuellement dans n'importe quel ordre, et qui s'effectuent finalement par un traitement thermique au moment de la réticulation des couches de mousse.

2. Corps constitué de couches composé d'une ou de plusieurs couches de mousse synthétique et/ou de couches en mousse de latex et de polymères superabsorbants en forme de particules d'après la revendication 1, caractérisé en ce que le rapport quantitatif de la couche de mousse synthétique et/ou de la couche de mousse de latex au polymère superabsorbant est de 1 : 50 à 25 : 1.

3. Corps constitué de couches composé d'une ou de plusieurs couches de mousse synthétique et/ou de couches en mousse de latex et de polymères superabsorbants en forme de particules d'après la revendication 1 ou 2, caractérisé en ce que le rapport quantitatif de la couche de mousse synthétique et/ou de la couche de mousse de latex au polymère superabsorbant est de 1 : 5 à 10 : 1.

4. Corps constitué de couches composé d'une ou de plusieurs couches de mousse synthétique et/ou de couches en mousse de latex et de polymères superabsorbants en forme de particules d'après une des revendications de 1 à 3 caractérisé en ce qu'il offre, pour une solution aqueuse de NaCI à 0,9 %,
- une rétention d'au moins 0,1 litre/m² de surface,
- une absorption maximale d'au moins 0,1 litre/m² de surface, et
- une absorption sous charge (AUL) d'au moins 2 g/g à 2,1 kPa.

5. Corps constitué de couches composé d'une ou de plusieurs couches de mousse synthétique et/ou de couches en mousse de latex et de polymères superabsorbants en forme de particules d'après une des revendications de 1 à 4, caractérisé en ce que les couches de mousse synthétique et/ou de mousse de couche en latex et/ou le polymère superabsorbant contiennent en plus au moins une matière de charge dont le taux de remplissage rapporté à la quantité de mousse synthétique et/ou de mousse en latex, est au maximum de 1000 % en poids, de préférence au maximum de 400 % en poids et, de manière optimale au maximum de 200 % en poids.

6. Corps constitué de couches composé d'une ou de plusieurs couches de mousse synthétique et/ou de couches en mousse de latex et de polymères superabsorbants en forme de particules d'après une des revendications de 1 à 5, caractérisé en ce que la couche de mousse synthétique et/ou la couche de mousse en latex et/ou le polymère superabsorbant contiennent, en tant que charge, de la craie, de la bentonite, des gels de silice, des acides siliques, du charbon actif, des pigments, de préférence de l'oxyde de titane et/ou de l'oxyde de fer, et/ou des matériaux en fibre naturelle ou synthétique, de préférence des fibres et des tissus de viscose et de coton et/ou des fibres de polyester et de polyamide, des mélanges de différentes fibres ou de différents tissus, et/ou de matières plastiques finement broyées.

7. Corps constitué de couches composé d'une ou de plusieurs couches de mousse synthétique et/ou de couches en mousse de latex et de polymères superabsorbants en forme de particules d'après une des revendications de 1 à 6, caractérisé en ce que la surface du corps comporte sur une partie, de préférence sur une face, une couche imperméable à l'eau et les liquides aqueux sous forme d'une feuille de plastique ou d'un tissu et/ou qu'elle comporte sur une partie, de préférence sur une face, une couche de cellulose, de voile cardé et/ou de papier et/ou un voile ou un tissu textile.

8. Procédé de fabrication d'un corps constitué de couches composé d'une ou de plusieurs couches de mousse synthétique et/ou de couches en mousse de latex et de polymères superabsorbants en forme de particules destiné à absorber l'eau et les liquides aqueux, caractérisé en ce qu'au moins
a) une mousse synthétique et/ou une mousse de latex d'une masse volumique de 50 à 1000 g/lit. est fabriquée et
la mousse est répartie de manière planéiforme à une épaisseur de couche de 1 µm à 100.000 µm, de préférence de 10 µm à 10.000 µm et d'une manière optimale de 200 µm à 5.000 µm,
b) le polymère superabsorbant formé de particules est appliqué avec un rapport quantitatif de la couche de mousse synthétique et/ou de mousse en latex au polymère superabsorbant de 1 : 500 à 50 : 1, de préférence de 1 : 50 à 25 : 1, et de manière optimale de 1 : 5 à 10 : 1 sur la mousse synthétique et/ou la mousse en latex répartie de manière planéiforme, en utilisant éventuellement au moins un moule, une grille et/ou un tamis en une répartition déterminée en quantité et surface, la couche de mousse synthétique et/ou de mousse en latex est vulcanisée par traitement thermique ou par traitement aux UV et le polymère superabsorbant est fixé, pour lequel l'(les) étape(s) de fabrication (a) et/ou (b) est (sont) éventuellement répétée(s) dans n'importe quel ordre, et un traitement thermique final est effectué au moment de la réticulation de (des) couche(s) de mousse.

9. Procédé de fabrication d'un corps constitué de couches composé d'une ou de plusieurs couches de mousse synthétique et/ou de couches en mousse de latex et de polymères superabsorbants en forme de particules d'après la revendication 8, caractérisé en ce que l'on utilise un polymère superabsorbant ayant une répartition granulométrique de 1 µm à 20.000 µm.

10. Procédé de fabrication d'un corps constitué de couches composé d'une ou de plusieurs couches de mousse synthétique et/ou de couches en mousse de latex et de polymères superabsorbants en forme de particules d'après les revendications 8 ou 9, caractérisé en ce que la fabrication s'effectue en utilisant des matériaux auxiliaires comme des surfaces de verre ou de métal, des feuilles de matière plastique et/ou du papier siliconé, desquels le corps constitué de couches est séparé après sa fabrication, ou en ce que la fabrication s'effectue à l'aide d'au moins une couche de cellulose, de voile cardé ou de papier et/ou d'un voile textile et/ou d'un tissu servant de couche intermédiaire, de couche de fond ou de couche finale de recouvrement.

11. Procédé de fabrication d'un corps constitué de couches composé d'une ou de plusieurs couches de mousse synthétique et/ou de couches en mousse de latex et de polymères superabsorbants en forme de particules d'après une des revendications de 8 à 10, caractérisé en ce que la quantité, la granulométrie, et la répartition du polymère superabsorbant en forme de particules sont différentes sur les diverses couches de mousse synthétique et/ou de mousse de latex.

12. Procédé de fabrication d'un corps constitué de couches composé d'une ou de plusieurs couches de mousse synthétique et/ou de couches en mousse de latex et de polymères superabsorbants en forme de particules d'après une des revendications de 8 à 11, caractérisé en ce qu'avant, pendant ou après la fabrication de la couche de mousse synthétique et/ou de mousse en latex, l'on introduit dans la mousse, en tant que charge, des bentonites, des gels de silice, des acides siliques, du charbon actif, des pigments minéraux, des matières plastiques broyées, de préférence de la craie et/ou des fibres naturelles ou synthétiques avec un taux de remplissage de 0 à 1000 % en poids rapporté à la mousse synthétique et/ou à la mousse de latex, de préférence à un taux maximal de 400 %, et un taux optimal au maximum de 200 %, éventuellement ensemble avec un ou plusieurs agents épaississants, un ou plusieurs agents de réticulation et un ou plusieurs agents stabilisants.

13. Procédé de fabrication d'un corps constitué de couches composé d'une ou de plusieurs couches de mousse synthétique et/ou de couches en mousse de latex et de polymères superabsorbants en forme de particules d'après une des revendications de 8 à 12, caractérisé en ce que la formation de la mousse s'effectue par mélange de deux ou plusieurs matières plastiques et/ou de latex et éventuellement en ajoutant des adjuvants comme des agents de moussage, des agents gonflants, des stabilisateurs de mousse, des agents de réticulation et des agents de vulcanisation.

14. Procédé de fabrication d'un corps constitué de couches composé d'une ou de plusieurs couches de mousse synthétique et/ou de couches en mousse de latex et de polymères superabsorbants en forme de particules d'après une des revendications de 8 à 13, caractérisé en ce que le corps constitué de couches est façonné pour la finition avec une calandre et/ou un rouleau de gaufrage.

15. Utilisation du corps constitué de couches d'après une des revendications de 1 à 7 dans les articles d'hygiène et dans le secteur sanitaire pour l'absorption de l'eau et des liquides corporels.

16. Utilisation du corps constitué de couches d'après la revendication 15 en tant que composant absorbant dans les langes pour bébé et dans les articles pour personnes incontinentes.

17. Utilisation du corps constitué de couches d'après une des revendications de 1 à 7 directement ou en tant que composant dans les sols naturels ou artificiels pour la culture des plantes ou pour le transport ou le stockage des plantes ou des parties de plantes.

18. Utilisation du corps constitué de couches d'après une des revendications de 1 à 7 en tant que matériau isolant pour les tubes et les conduites, de préférence pour les câbles électriques et les câbles à fibres optiques.

19. Utilisation du corps constitué de couches d'après une des revendications de 1 à 7 en tant que matériau isolant pour les constructions de bâtiments, de préférence pour les murs extérieurs.

20. Utilisation du corps constitué de couches d'après une des revendications de 1 à 7 directement ou en tant que composant absorbant les liquides et/ou emmagasinant les liquides dans les matériaux d'emballage.

21. Utilisation du corps constitué de couches d'après une des revendications de 1 à 7 en tant que constituant dans les pièces d'habillage.
